(19) Europäisches Patentamt
European Patent Office
Office européen des brevets

(11) EP 2 981 546 B1

(12) **EUROPEAN PATENT SPECIFICATION**

(45) Date of publication and mention of the grant of the patent:
**11.01.2017 Bulletin 2017/02**

(21) Application number: **14715280.5**

(22) Date of filing: **03.04.2014**

(51) Int Cl.:
***C07K 14/38*** *(2006.01)*

(86) International application number:
**PCT/EP2014/056689**

(87) International publication number:
**WO 2014/161936 (09.10.2014 Gazette 2014/41)**

(54) **A FILAMENTOUS FUNGAL CELL WITH INACTIVATED COMPONENT OF THE SELECTIVE AUTOPHAGY PATHWAY AND METHOD OF USING SAME**

FILAMENTÖSE PILZZELLE MIT INAKTIVIERTER KOMPONENTE DES SELEKTIVEN AUTOPHAGIESIGNALWEGS UND VERFAHREN ZUR VERWENDUNG DAVON

CELLULE FONGIQUE FILAMENTEUSE AYANT UN COMPOSÉ INACTIVÉ DE LA VOIE D'AUTOPHAGIE SÉLECTIVE, ET PROCÉDÉ D'UTILISATION ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.04.2013 US 201361807980 P**

(43) Date of publication of application:
**10.02.2016 Bulletin 2016/06**

(73) Proprietor: **Novozymes A/S**
**2880 Bagsvaerd (DK)**

(72) Inventor: **UDAGAWA, Hiroaki**
**Chiba 272-0035 (JP)**

(56) References cited:
**US-B1- 6 432 914**

- **XIAO-HONG LIU ET AL: "Investigation of the biological roles of autophagy in appressorium morphogenesis in Magnaporthe oryzae", JOURNAL OF ZHEJIANG UNIVERSITY SCIENCE B, vol. 9, no. 10, 1 October 2008 (2008-10-01), pages 793-796, XP055123856, ISSN: 1673-1581, DOI: 10.1631/jzus.B0860013**
- **SHINYA KIMURA ET AL: "Autophagy delivers misfolded secretory proteins accumulated in endoplasmic reticulum to vacuoles in the filamentous fungus", BIOCHEMICAL AND BIOPHYSICAL RESEARCH COMMUNICATIONS, vol. 406, no. 3, 2011, - 2011, pages 464-470, XP028166950, ISSN: 0006-291X, DOI: 10.1016/J.BBRC.2011.02.075 [retrieved on 2011-02-18]**
- **IRSHAD ALI KHAN ET AL: "Multifunction of autophagy-related genes in filamentous fungi", MICROBIOLOGICAL RESEARCH, FISCHER, JENA, DE, vol. 167, no. 6, 16 January 2012 (2012-01-16), pages 339-345, XP028486582, ISSN: 0944-5013, DOI: 10.1016/J.MICRES.2012.01.004 [retrieved on 2012-01-26]**
- **POLLACK J K ET AL: "Autophagy in filamentous fungi", FUNGAL GENETICS AND BIOLOGY, SAN DIEGO, CA, US, vol. 46, no. 1, 1 January 2009 (2009-01-01), pages 1-8, XP025805234, ISSN: 1087-1845, DOI: 10.1016/J.FGB.2008.10.010 [retrieved on 2008-11-05]**



Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

## Description

### Reference to sequence listing

This application contains a Sequence Listing in computer readable form.

### FIELD OF THE INVENTION

The invention relates to modified filamentous fungal cells and to methods for producing biological compounds, such as, a polypeptides (e.g., enzymes), using such modified filamentous fungal cells.

### BACKGROUND OF THE INVENTION

Autophagy is a system in eukaryotic cells in which cytoplasmic components and organelles are digested using vacuoles/lysosomes. Autophagy has been linked to nutrient recycling during starvation as well as in growth and development as well as other cellular events. The mechanism of autophagy is complex, and may involve the interaction of more than 30 autophagy genes designated as atg1, atg2, atg3, atg4, atg5, atg6, atg7, atg8, atg9, atg10, atg11, atg12, atg13, atg14, atg15, atg16, atg17, atg18, atg19, atg20, atg21, atg22, atg23, atg24, atg25, atg26, atg27, atg28, atg29, atg30, and atg31 genes.

Autophagy and the role of many of the autophagy genes have been studied in a number of eukaryotic cells, including in yeast and filamentous fungi. Marten et al., "Autophagy in filamentous fungi," Fungal Genetics and Biology, 46 (2009), 1-8; Askew et al., "Unexpected link between Metal Ion Deficiency and Autophagy in Aspergillus fumigatus," Eukaryotic Cell, Dec. 2007, 2437-2447; Kitamoto et al., "Functional anaylysis of the ATG8 Homologoue Aoatg8 and Role of Autophagy in Differentiation and Germination of Aspergillus oryzae," Eukaryotic Cell, Aug. 2006, 1328-1336; Kitamoto K. & Kikuma T., "Analysis of autophagy in Aspergillus oryzae by disruption of Aoatg13, Aoatg4, and Aoatg15 genes," FEMS Microbiol Lett 316 (2011) 61-69; Klionsky et al., "The Atg1 Kinase Complex Is Involved in the Regulation of Protein Recruitment to Initiate Sequestering Vesicle Formation for Nonspecific Autophagy in Saccharomyces cerevisiaes," Molecular Biology of the Cell, Vol. 19, Feb. 2008, 668-681; van der Klei, "Autophagy Deficiency Promotes β-Lactam Production in Penicilium chrysogenum," Applied and Environmental Microbiology, Vol. 77, No. 4, Feb. 2011, 1413-1422; Klionsky et al., "The Actin Cytoskeleton Is Required For Selective Types of Autophagy, but Not Nonspecific Autophagy, in the Yeast Saccharomyces cerevisiae," Molecular Biology of the Cell, Vol. 16, Dec. 2005, 5843-5865; Klionsky D. J. & Cheong H., "Dual role of Atg1 in regulation of autophagy-specific PAS assembly in Saccharomyces cerevisiae," Autophagy, 4:5, July 2008, 724-726; Chung et al., "ATG1, and autophagy regulator, inhibit cell growth by negatively regulating S6 kinase," EMBO reports, Vol. 8, No. 4, (2007), 360-365; Mizushima, "The role of the atg1/ULK1 complex in autophagy regulation," Current Opinion in Cell Biology, 22:132-139 (2010); Kitamoto et al., "Functional analysis of Aoatg1 and detection of the Cvt pathway in Aspergillus oryzae," (2012); Klionsky D. & Yorimitsu T., "Atg11 Links Cargo to the Vesicle-forming Machinery in the Cytoplasm to Vaculoe Targeting Pathway," Molecular Biology of the Cell, Vol. 16, April 2005, 159301605; and K. Suzuki, "Selective autophagy in budding yeast," Cell Death and Differentiation (2013) 20, 43-48.

The complete role of the autophagy system and the autophagy genes, however, has not been fully elucidated.

### SUMMARY OF THE INVENTION

The invention is directed to genetically modified filamentous fungal host cells in which the selective autophagy system of a filamentous fungal cell has been partially or completely inactivated, and the production of polypeptides in such modified host cells. The inactivation of the selective autophagy system of a filamentous fungal cell results in increased expression yield of a biological product of interest, such as, a polypeptide of interest. In a particular embodiment, the invention provides a modified filamentous fungal host cell comprising a partially or completely inactivated selective autophagy system, wherein the modified filamentous fungal host cell has been transformed with a gene encoding a biological product of interest, such as, a gene encoding a polypeptide (e.g., an enzyme) of interest. The activation of the selective autophagy system in a filamentous fungal cell may be accomplished by deleting or disrupting one or more genes/proteins (atg genes/proteins) involved in the selective autophagy pathway of the filamentous fungal cell, including by deleting, disrupting, or silencing the endogenous atg11 activity present in the filamentous fungal cell and/or by deleting or disrupting one or more genes/proteins that are involved directly or indirectly with atg11 activity in the selective autophagy pathway of the filamentous fungal cell.

The invention is directed to genetically modified filamentous fungal host cells in which the atg11 activity of a filamentous fungal cell has been deleted, disrupted or silenced, such as, by deleting or disrupting the expression of endogenous atg11 gene present in the filamentous fungal cell. The deletion, disruption or silencing of the atg11 activity in filamentous fungal host cells results in increased expression yield of a biological product of interest, such as, a polypeptide of interest. In a particular embodiment, the invention provides a modified filamentous fungal host cell comprising a deleted or disrupted or silenced atg11 gene, which filamentous fungal host cell has been transformed with a gene encoding a biological product of interest, such as, a gene encoding a polypeptide (e.g., an enzyme) of interest.

The invention also provides methods for producing modified filamentous fungal host cells in which the atg11 activity of the fungal cell has been deleted or disrupted or silenced. In a particular embodiment, the invention provides a method for producing a filamentous fungal host cell comprising deleting or disrupting the atg11 gene in the filamentous fungal cell. In another embodiment, the invention provides a method for producing a filamentous fungal host cell comprising transforming a filamentous fungal cell with an expression vector encoding a biological product of interest, such as a polypeptide of interest, and wherein the filamentous fungal cell has been modified or is further modified to comprise a deletion or disruption or silencing of the atg11 gene.

The invention further provides methods for producing a biological compound of interest, including recombinant production of a polypeptide of interest, using modified filamentous fungal host cells in which the atg11 activity of the fungal cell has been deleted or disrupted. In a particular embodiment, the present invention comprises culturing a filamentous fungal cell comprising a gene encoding a polypeptide of interest, wherein the atg11 gene of the fungal cell has been deleted or disrupted. The culturing occurs under conditions conducive for inducing expression of the heterologous polypeptide of interest, which may be recovered from the culture medium for use in one more applications or alternatively the culture medium or fermentation broth containing the polypeptide of interest may be used in one more applications.

The host cells and methods of the present invention may be applied to produce any biological product of interest. In particular embodiments, the modified filamentous host cells and methods are applied to produce a polypeptide of interest, such as, a hormone, enzyme, receptor or portion thereof, antibody or portion thereof, or a reporter. Particular embodiments of the invention are directed to using the modified filamentous fungal host cells and methods of the present invention in recombinantly producing an enzyme of interest.

## DETAILED DESCRIPTION OF THE INVENTION

### Definitions

**Atg11:** The term "atg11" refers to the autophagy related protein that is believed to be an adapter protein involved in selective autophagy. Examples of atg11 proteins and encoding nucleic acid sequences are known in the art and include, for example, the *Aspgerillus niger* atg11 protein having the having the amino acid of SEQ ID NO:2, as encoded by the nucleic acid sequence shown in SEQ ID NO:1. Other atg11 proteins and genes have been characterized, such as, for example, in *Aspergillus oryzae* and *Aspergillus nidulans.* As atg11 genes and proteins are structurally and functionally similar in fungal cells, other atg11 genes and proteins (atg11 gene and protein homologs) can identified in other fungal hosts using routine molecular biological methods. One skilled in the art will also realize that variations in atg11 sequences can occur in different cell lines that will not alter the protein function.

**Biological product:** The term "biological product" refers to any product of interest produced in a filamentous fungal host cells. The biological product may be the expression product which yield is directly increased by the partial or complete inactivation of the selective autophagy pathway, such as, by the deletion or disruption of the atg11, or a product which is created from the action of an expression product that is directly increased by the inactivation of the selective autophagy pathway, such as, by the atg11 activity disruption or deletion.

**cDNA:** The term "cDNA" means a DNA molecule that can be prepared by reverse transcription from a mature, spliced, mRNA molecule obtained from a eukaryotic or prokaryotic cell. cDNA lacks intron sequences that may be present in the corresponding genomic DNA. The initial, primary RNA transcript is a precursor to mRNA that is processed through a series of steps, including splicing, before appearing as mature spliced mRNA.

**Coding sequence:** The term "coding sequence" means a polynucleotide, which directly specifies the amino acid sequence of a polypeptide. The boundaries of the coding sequence are generally determined by an open reading frame, which begins with a start codon such as ATG, GTG, or TTG and ends with a stop codon such as TAA, TAG, or TGA. The coding sequence may be a genomic DNA, cDNA, synthetic DNA, or a combination thereof.

**Control sequences:** The term "control sequences" means nucleic acid sequences necessary for expression of a polynucleotide encoding a mature polypeptide. Each control sequence may be native (*i.e.*, from the same gene) or foreign (*i.e.*, from a different gene) to the polynucleotide encoding the polypeptide or native or foreign to each other. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the polynucleotide encoding a polypeptide. The term "control sequences" is defined herein to include all components, which are necessary or advantageous for the expression of a polypeptide.

The control sequence may be an appropriate promoter sequence, a nucleic acid sequence which is recognized by a host cell for expression of the nucleic acid sequence. The promoter sequence contains transcriptional control sequences which mediate the expression of the polypeptide. The promoter may be any nucleic acid sequence which shows transcriptional activity in the host cell of choice including mutant, truncated, and hybrid promoters, and may be obtained from genes encoding extracellular or intracellular polypeptides either homologous or heterologous to the host cell.

Examples of suitable promoters for directing the transcription of the nucleic acid constructs in a filamentous fungal host cell are promoters obtained from the genes for *Aspergillus oryzae* TAKA amylase, *Rhizomucor miehei* aspartic proteinase, *Aspergillus niger* neutral alpha-amylase, *Aspergillus niger* acid stable alpha-amylase, *Aspergillus niger* or *Aspergillus awamori* glucoamylase (glaA), *Rhizomucor miehei* lipase, *Aspergillus oryzae* alkaline protease, *Aspergillus oryzae* triose phosphate isomerase, *Aspergillus nidulans* acetamidase, *Fusarium venenatum* amyloglucosidase, *Fusarium oxysporum* trypsin-like protease (WO 96/00787), as well as the NA2-tpi promoter (a hybrid of the promoters from the genes for *Aspergillus niger* neutral alpha-amylase and *Aspergillus oryzae* triose phosphate isomerase); and mutant, truncated, and hybrid promoters thereof.

The control sequence may be a suitable transcription terminator sequence, a sequence recognized by a host cell to terminate transcription. The terminator sequence is operably linked to the 3' terminus of the nucleic acid sequence encoding the polypeptide. Any terminator which is functional in the host cell of choice may be used in the present invention.

Preferred terminators for filamentous fungal host cells are obtained from the genes for *Aspergillus oryzae* TAKA amylase, *Aspergillus niger* glucoamylase, *Aspergillus nidulans* anthranilate synthase, *Aspergillus niger* alpha-glucosidase, and *Fusarium oxysporum* trypsin-like protease.

The control sequence may also be a suitable leader sequence, a non-translated region of an mRNA which is important for translation by the host cell. The leader sequence is operably linked to the 5' terminus of the nucleic acid sequence encoding the polypeptide. Any leader sequence that is functional in the host cell of choice may be used in the present invention, such as, for example, the genes for *Aspergillus oryzae* TAKA amylase and *Aspergillus nidulans* triose phosphate isomerase. Mcknight G. L., O'Hara P. J., Parker M. L., "Nucleotide sequence of the triosephosphate isomerase gene from Aspergillus nidulans: Implications for a differential loss of introns", Cell 46:143-147 (1986)).

The control sequence may also be a polyadenylation sequence, a sequence operably linked to the 3' terminus of the nucleic acid sequence and which, when transcribed, is recognized by the host cell as a signal to add polyadenosine residues to transcribed mRNA. Any polyadenylation sequence which is functional in the host cell of choice may be used in the present invention. Examples include the polyadenylation sequences for the genes for *Aspergillus oryzae* TAKA amylase, *Aspergillus niger* glucoamylase, *Aspergillus nidulans* anthranilate synthase, *Fusarium oxysporum* trypsin-like protease, and *Aspergillus niger* alpha-glucosidase.

The control sequence may also be a signal peptide coding region that codes for an amino acid sequence linked to the amino terminus of a polypeptide and directs the encoded polypeptide into the cell's secretory pathway. The 5' end of the coding sequence of the nucleic acid sequence may inherently contain a signal peptide coding region naturally linked in translation reading frame with the segment of the coding region which encodes the secreted polypeptide. Alternatively, the 5' end of the coding sequence may contain a signal peptide coding region which is foreign to the coding sequence. The foreign signal peptide coding region may be required where the coding sequence does not naturally contain a signal peptide coding region. Alternatively, the foreign signal peptide coding region may simply replace the natural signal peptide coding region in order to enhance secretion of the polypeptide. However, any signal peptide coding region which directs the expressed polypeptide into the secretory pathway of a host cell of choice may be used in the present invention.

Effective signal peptide coding regions for filamentous fungal host cells are the signal peptide coding regions obtained from the genes for *Aspergillus oryzae* TAKA amylase, *Aspergillus niger* neutral amylase, *Aspergillus niger* glucoamylase, *Rhizomucor miehei* aspartic proteinase, *Humicola insolens* cellulase, and *Humicola lanuginosa* lipase.

The control sequence may also be a propeptide coding region that codes for an amino acid sequence positioned at the amino terminus of a polypeptide. The resultant polypeptide is known as a proenzyme or propolypeptide (or a zymogen in some cases). A propolypeptide is generally inactive and can be converted to a mature active polypeptide by catalytic or autocatalytic cleavage of the propeptide from the propolypeptide. The propeptide coding region may be obtained from the genes for *Bacillus subtilis* alkaline protease (aprE), *Bacillus subtilis* neutral protease (nprT), *Saccharomyces cerevisiae* alpha-factor, *Rhizomucor miehei* aspartic proteinase, and *Myceliophthora thermophila* laccase (WO 95/33836).

Where both signal peptide and propeptide regions are present at the amino terminus of a polypeptide, the propeptide region is positioned next to the amino terminus of a polypeptide and the signal peptide region is positioned next to the amino terminus of the propeptide region.

**Deleted or disrupted:** The term "deleted or disrupted" (and "deletion or disruption") refers to the elimination of or reduction of the protein function or activity in a modified host cell relative to a host cell of the same species which does not comprise the modification to the gene or protein being assessed. The reduction of the protein function or activity of the atg11 protein (or other components of the selective autophagy pathway) is sufficient if it enables the increased yield of a protein in a fungal host. The deletion or disruption of the atg11 protein function or activity (or other components of the selective autophagy pathway) may be obtained by 1) deletion or disruption of the upstream or downstream regulatory sequences controlling expression (e.g., of the atg11 gene or atg11 gene homolog) and 2) mutation of a gene (e.g., gene encoding the atg11 gene protei)n to render the gene non-functional, wherein "mutation" includes deletion, substitution, insertion or addition into the gene to render the encoded protein incapable of having the activity or function.

**Expression:** The term "expression" includes any step involved in the production of a polypeptide including, but not limited to, transcription, post-transcriptional modification, translation, post-translational modification, and secretion.

**Expression vector:** The term "expression vector" means a linear or circular DNA molecule that comprises a polynucleotide encoding a polypeptide and is operably linked to control sequences that provide for its expression.

**Filamentous Fungal:** The term "filamentous fungal" (or "filamentous fungi") refers to any filamentous fungal host cells, and includes all filamentous forms of the subdivision Eumycota and Oomycota (as defined by Hawksworth et al., 1995, supra). The filamentous fungi are characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative. In a preferred embodiment, the filamentous fungal host cell is a cell of a species of, but not limited to, *Acremonium, Aspergillus, Fusarium, Humicola, Mucor, Myceliophthora, Neurospora, Penicillium, Thielavia, Tolypocladium,* or *Trichoderma.* In an embodiment, the filamentous fungal host cell is an *Aspergillus awamori, Aspergillus foetidus, Aspergillus japonicus, Aspergillus nidulans, Aspergillus niger* or *Aspergillus oryzae* cell. In another embodiment, the filamentous fungal host cell is a *Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides,* or *Fusarium venenatum* cell. In another embodiment, the filamentous fungal host cell is a *Humicola insolens, Humicola lanuginosa, Mucor miehei, Myceliophthora thermophila, Neurospora crassa, Penicillium purpurogenum, Thielavia terrestris, Trichoderma harzianum, Trichoderma koningii, Trichoderma longibrachiatum, Trichoderma reesei,* or *Trichoderma viride* cell.

**Heterologous:** The polypeptide of interest may be native or heterologous to the filamentous fungal host cell of interest. The term "heterologous polypeptide" is defined herein as a polypeptide which is not native to the fungal cell, a native polypeptide in which modifications have been made to alter the native sequence, or a native polypeptide whose expression is quantitatively altered as a result of a manipulation of the fungal cell by recombinant DNA techniques. The polynucleotide encoding the polypeptide of interest may originate from any organism capable of producing the polypeptide of interest, including multicellular organisms and microorganisms, e.g. bacteria and fungi.

**Homolog:** The term "homolog" refers to a gene or protein sequence that shares structural and functional similarity to a reference sequence. The term "homolog" includes both orthologs, which are sequences in different species that are structurally similar due to evolution from a common ancestor, and paralogs, which are similar sequences within the same genome.

**Inactivated.** The term "inactivated" (or "inactivation") refers to the partial or complete elimination of the activity in a modified host cell relative to a host cell of the same species which does not comprise the modification to the gene or protein being assessed.

**Increased yield:** The term "increased yield" means increased capacity for the modified fungal cell to produce a polypeptide of interest relative to a host cell of the same species which does not comprise the modification (such as, to the atg11 gene or protein or other gene or protein being assessed). In accordance with the present invention, the cell produces more of a polypeptide of interest relative to a host cell of the same species which does not comprise the modification in the same amount of time.

**Introduction:** The term "introduction" means introducing a vector comprising the nucleic acid sequence encoding the polypeptide into a fungal cell so that the vector is maintained as a chromosomal integrant or as a self-replicating extra-chromosomal vector. Integration is generally considered to be an advantage as the nucleic acid sequence is more likely to be stably maintained in the cell. Integration of the vector into the chromosome occurs by homologous recombination, non-homologous recombination, or transposition. For integration into the host cell genome, the vector may rely on the nucleic acid sequence encoding the polypeptide or any other element of the vector for stable integration of the vector into the genome by homologous or nonhomologous recombination. Alternatively, the vector may contain additional nucleic acid sequences for directing integration by homologous recombination into the genome of the host cell. The additional nucleic acid sequences enable the vector to be integrated into the host cell genome at a precise location(s) in the chromosome(s). To increase the likelihood of integration at a precise location, the integrational elements should preferably contain a sufficient number of nucleic acids, such as 100 to 1,500 base pairs, preferably 400 to 1,500 base pairs, and most preferably 800 to 1,500 base pairs, which are highly homologous with the corresponding target sequence to enhance the probability of homologous recombination. The integrational elements may be any sequence that is homologous with the target sequence in the genome of the host cell. These nucleic acid sequences may be any sequence that is homologous with a target sequence in the genome of the host cell, and, furthermore, may be non-encoding or encoding sequences. For autonomous replication, the vector may further comprise an origin of replication enabling the vector to replicate autonomously in the host cell. The procedures used to ligate the elements described above to construct the recombinant expression vectors are well known to one skilled in the art (see, e.g., Sambrook, et al., supra).

**Nucleic acid construct**: The term "nucleic acid construct" is defined herein as a nucleic acid molecule, either single- or double-stranded, which is isolated from a naturally occurring gene or which has been modified to contain segments of nucleic acid which are combined and juxtaposed in a manner which would not otherwise exist in nature. The term nucleic acid construct is synonymous with the term expression cassette when the nucleic acid construct contains all the control sequences required for expression of a coding sequence. The term "coding sequence" as defined herein is a sequence, which is transcribed into mRNA and translated into a transcriptional activator of the present invention. The boundaries of the coding sequence are generally determined by the ATG start codon at the 5' end of the mRNA and a transcription terminator sequence located just downstream of the open reading frame at the 3' end of the mRNA. A coding sequence can include, but is not limited to, DNA, cDNA, and recombinant nucleic acid sequences.

**Polypeptide of interest:** The term "polypeptide of interest" is not meant herein to refer to a specific length of the encoded product and, therefore, encompasses peptides, oligopeptides, and proteins. Wherein the polypeptide is not naturally secreted, the nucleic acid encoding the protein may be modified to have a signal sequence in accordance with techniques known in the art. The polypeptides which are secreted may be endogenous polypeptides which are expressed naturally, but in a greater amount in accordance with the present invention, or the polypeptides may be heterologous. In a preferred embodiment, the polypeptides are heterologous. Therefore, the polypeptide may be native to the cell, but is produced, for example, by transformation with a self-replicating vector containing the nucleic acid encoding the polypeptide of interest. Alternatively, recombinant could be wherein one or more extra copies of the nucleic acid are integrated into the genome by recombinant techniques. In embodiment, the protein of interest is selected from the group consisting of peptidase (carboxypeptidase, aminopepetidase, protease), amylase (e.g., alpha-amylase or glucoamylase), carbohydrase, catalase, cellulase, chitinase, cutinase, cyclodextrin glycosyltransferase, deoxyribonuclease, esterase, endo-glucosidase, alpha-galactosidase, beta-galactosidase, alpha-glucosidase, beta-glucosidase, invertase, laccase, lipase, phospholipase, mannosidase, mutanase, oxidase, pectinolytic enzyme, peroxidase, phytase, polyphenoloxidase, ribonuclease, transglutaminase, and xylanase. In another embodiment, the protein of interest is a therapeutic selected from the group consisting of vaccines, cytokines, receptors, antibodies, hormones, and factors including growth factors. In a preferred embodiment, the polypeptide of interest is secreted extracellularly.

The nucleic acid sequence encoding a polypeptide of interest may be obtained from any prokaryotic, eukaryotic, or other source. For purposes of the present invention, the term "obtained from" as used herein in connection with a given source shall mean that the polypeptide is produced by the source or by a cell in which a gene from the source has been inserted.

The techniques used to isolate or clone a nucleic acid sequence encoding a polypeptide of interest are known in the art and include isolation from genomic DNA, preparation from cDNA, or a combination thereof. The cloning of the nucleic acid sequence from such genomic DNA can be effected, e.g., by using the well-known polymerase chain reaction (PCR). See, for example, Innis et al., 1990, PCR Protocols: A Guide to Methods and Application, Academic Press, New York. The cloning procedures may involve excision and isolation of a desired nucleic acid fragment comprising the nucleic acid sequence encoding the polypeptide, insertion of the fragment into a vector molecule, and incorporation of the recombinant vector into the mutant fungal cell where multiple copies or clones of the nucleic acid sequence will be replicated. The nucleic acid sequence may be of genomic, cDNA, RNA, semisynthetic, synthetic origin, or any combinations thereof.

**Recombinant expression vector:** The term "recombinant expression vector" may be any vector (e.g., a plasmid or virus), which can be conveniently subjected to recombinant DNA procedures and can bring about the expression of the nucleic acid sequence encoding the polypeptide. The choice of the vector will typically depend on the compatibility of the vector with the fungal cell into which the vector is to be introduced. The vectors may be linear or closed circular plasmids. The vector may be an autonomously replicating vector, i.e., a vector, which exists as an extrachromosomal entity, the replication of which is independent of chromosomal replication, e.g., a plasmid, an extrachromosomal element, a minichromosome, or an artificial chromosome. The vector may contain any means for assuring self-replication. Alternatively, the vector may be one which, when introduced into the filamentous fungal cell, is integrated into the genome and replicated together with the chromosome(s) into which it has been integrated. The vector system may be a single vector or plasmid or two or more vectors or plasmids, which together contain the total DNA to be introduced into the genome of the fungal cell, or a transposon.

The vectors preferably contain one or more selectable markers, which permit easy selection of transformed cells. A selectable marker is a gene the product of which provides for biocide or viral resistance, resistance to heavy metals, prototrophy to auxotrophs, and the like. A selectable marker for use in a filamentous fungal cell may be selected from the group including, but not limited to, amdS (acetamidase), argB (ornithine carbamoyltransferase), bar (phosphinothricin acetyltransferase), hygB (hygromycin phosphotransferase), niaD (nitrate reductase), pyrG (orotidine-5'-phosphate decarboxylase), sC (sulfate adenyltransferase), and trpC (anthranilate synthase), as well as equivalents from other species. Preferred for use in an *Aspergillus* cell are the amdS and pyrG genes of *Aspergillus nidulans* or *Aspergillus oryzae* and the bar gene of *Streptomyces hygroscopicus*. The vectors preferably contain an element(s) that permits stable integration of the vector into the host cell genome or autonomous replication of the vector in the cell independent of the genome of the cell.

The introduction of an expression vector or a nucleic acid construct into a fungal cell may involve a process consisting of protoplast formation, transformation of the protoplasts, and regeneration of the cell wall in a manner known per se. Suitable procedures for transformation of *Aspergillus* cells are described in EP 238 023 and Yelton et al., 1984, Proceedings of the National Academy of Sciences USA 81: 1470-1474. A suitable method of transforming Fusarium species is described by Malardier et al., 1989, Gene 78: 147-156 or in WO 96/00787.

**Recombinant nucleic acid:** The term "recombinant nucleic acid" means nucleic acid, originally formed in vitro, in general, by the manipulation of nucleic acid by polymerases and endonucleases, in a form not normally found in nature. Generally, a nucleic acid refers to DNA, RNA or mRNA and includes a gene or gene fragment. Thus, an isolated nucleic acid, in a linear form, or an expression vector formed in vitro by ligating DNA molecules that are not normally joined, are both considered recombinant for the purposes of this invention. It is understood that once a recombinant nucleic acid is made and reintroduced into a host cell or organism, it will replicate non-recombinantly, i.e. using the in vivo cellular machinery of the host cell rather than in vitro manipulations; however, such nucleic acids, once produced recombinantly, although subsequently replicated non-recombinantly, are still considered recombinant for the purposes of the invention.

**Recombinant protein:** The term "recombinant protein" is a protein made using recombinant techniques, i.e. through the expression of a recombinant nucleic acid as depicted above. Generally, the term protein and peptide or polypeptide can be used interchangeably herein. A recombinant protein is distinguished from naturally occurring protein by at least one or more characteristics. For example, the protein may be isolated or purified away from some or all of the proteins and compounds with which it is normally associated in its wild type host, and thus may be substantially pure. For example, an isolated protein is unaccompanied by at least some of the material with which it is normally associated in its natural state, preferably constituting at least about 0.5%, more preferably at least about 5% by weight of the total protein in a given sample. A substantially pure protein comprises at least about 75% by weight of the total protein, with at least about 80% being preferred, and at least about 90% being particularly preferred. In one embodiment, the definition includes the production of a protein from other than its host cell, or produced by a recombinant nucleic acid. Alternatively, the protein may be made at a significantly higher concentration than is normally seen, through the use of an inducible promoter or high expression promoter, such that the protein is made at increased concentration levels. Alternatively, the protein may be in a form not normally found in nature, as in the addition of an epitope tag or amino acid substitutions, insertions and deletions, as discussed below.

**Recombinant cell:** The term "recombinant cell" generally refers to a cell which has been manipulated to contain a recombinant nucleic acid or polypeptide (protein) therein. Transfer of the genes into these cells can be achieved, for instance, by using the conventional methods of transformation described for these organisms. General aspects of mammalian cell host system transfections have been described in U.S. Pat. No. 4,399,216. Transformations into yeast are typically carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, etc. For various techniques for transforming mammalian cells, see Keown et al., Methods in Enzymology, 185:527-537 (1990) and Mansour et al., Nature, 336:348-352 (1988).

The nucleic acid (e.g., cDNA, coding or genomic DNA) encoding the UPR modulating protein may be inserted into a replicable vector. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

**Selectable marker:** The term "selectable marker" is defined herein as a gene the product of which provides for biocide or viral resistance, resistance to heavy metals, prototrophy to auxotrophs, and the like, which permits easy selection of transformed cells. Selectable markers for use in a filamentous fungal host cell include, but are not limited to, amdS (acetamidase), argB (ornithine carbamoyltransferase), bar (phosphinothricin acetyltransferase), hygB (hygromycin phosphotransferase), niaD (nitrate reductase), pyrG (orotidine-5'-phosphate decarboxylase), sC (sulfate adenyltransferase), trpC (anthranilate synthase), as well as equivalents thereof. Preferred for use in an *Aspergillus* cell are the amdS and pyrG genes of *Aspergillus nidulans* or *Aspergillus oryzae* and the bar gene of *Streptomyces hygroscopicus*. Functional derivatives of these selectable markers are also of interest in the present invention, in particular those functional derivatives which have decreased activity or decreased stability, thereby enabling a selection for a higher copy-number of the expression vector without increasing the concentration of the selective substance(s).

**Selective autophagy:** The term "selective autophagy" refers to the autophagy system which is dependent on the atg11 activity in the filamentous fungal cell.

**Sequence identity:** The relatedness between two amino acid sequences or between two nucleotide sequences is described by the parameter "sequence identity". For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

(Identical Residues x 100)/(Length of Alignment – Total Number of Gaps in Alignment)

For purposes of the present invention, the sequence identity between two deoxyribonucleotide sequences is determined using the Needleman-Wunsch algorithm (Needleman and Wunsch, 1970, *supra*) as implemented in the Needle program of the EMBOSS package (EMBOSS: The European Molecular Biology Open Software Suite, Rice *et al.*, 2000, *supra*), preferably version 5.0.0 or later. The parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

(Identical Deoxyribonucleotides x 100)/(Length of Alignment – Total Number of Gaps in Alignment)

## DETAILED DESCRIPTION OF THE INVENTION

The invention provides genetically modified filamentous fungal cells in which the selective autophagy system of the filamentous fungal cell has been partially or completely inactivated, methods of making such genetically modified filamentous fungal cells, and methods for producing biological products of interest in such modified filamentous fungal cells.

The selective autophagy system of a filamentous fungal cell may be inactivated (partially or completely) by the deletion or disruption of particular genes/proteins involved in the selective autophagy pathway. In a particular embodiment, the inactivation of the selective autophagy system in a filamentous fungal cell may be accomplished by deleting or disrupting the endogenous atg11 activity present in the filamentous fungal cell. In another embodiment, the inactivation of the selective autophagy system in a filamentous fungal cell may be accomplished by deleting or disrupting one or more genes/proteins of the selective autophagy pathway of the filamentous fungal cell that are involved directly or indirectly with atg11 activity in the selective autophagy pathway of the filamentous fungal cell.

The present invention provides genetically modified filamentous fungal cells comprising disrupted or deleted atg11 activity, methods of making such genetically modified filamentous fungal cells, and methods for producing biological products of interest in such modified filamentous fungal cells.

In accordance with the present invention a modified filamentous fungal cell is obtained by disrupting or deleting the endogenous atg11 gene, or a control sequence thereof, which results in the modified fungal cell producing none or less of the polypeptide having atg11 activity. The atg11 deficient mutant cells created are particularly useful as host cells for the expression of homologous and/or heterologous expression products, such as homologous or heterologous polypeptides or primary or secondary metabolites. Therefore, the present invention further relates to methods for producing a homologous or heterologous product comprising (a) cultivating the modified fungal cell under conditions conducive for production of the product; and (b) recovering the product.

The construction of strains that have reduced atg11 activity may be conveniently accomplished by modification or inactivation of a nucleic acid sequence necessary for expression of the atg11 polypeptide in the cell. The nucleic acid sequence to be modified or inactivated may be, for example, a nucleic acid sequence encoding the polypeptide or a part thereof essential for exhibiting atg11 activity, e.g., the nucleic acid sequenced of SEQ ID NO:1 or a part thereof, or a nucleic acid sequence which may have a regulatory function required for the expression of the polypeptide from the coding sequence of the nucleic acid sequence. An example of such a regulatory or control sequence may be a promoter sequence or a functional part thereof, i.e., a part which is sufficient for affecting expression of the polypeptide. Other control sequences for possible modification are described further in this section.

Modification or inactivation of the nucleic acid sequence may be performed by subjecting the cell to mutagenesis and selecting or screening for cells in which the atg11 activity has been reduced or eliminated. The mutagenesis, which may be specific or random, may be performed, for example, by use of a suitable physical or chemical mutagenizing agent, by use of a suitable oligonucleotide, or by subjecting the DNA sequence to PCR generated mutagenesis. Furthermore, the mutagenesis may be performed by use of any combination of these mutagenizing agents.

Examples of a physical or chemical mutagenizing agent suitable for the present purpose include ultraviolet (UV) irradiation, hydroxylamine, N-methyl-N'-nitro-N-nitrosoguanidine (MNNG), O-methyl hydroxylamine, nitrous acid, ethyl methane sulphonate (EMS), sodium bisulphite, formic acid, and nucleotide analogues. When such agents are used, the mutagenesis is typically performed by incubating the cell to be mutagenized in the presence of the mutagenizing agent of choice under suitable conditions, and selecting for cells exhibiting reduced atg11 activity or production.

Modification or inactivation of production of atg11 activity of the fungal cell may be particularly accomplished by introduction, substitution, or removal of one or more nucleotides in the nucleic acid sequence encoding the polypeptide or a regulatory element required for the transcription or translation thereof. For example, nucleotides may be inserted or removed so as to result in the introduction of a stop codon, the removal of the start codon, or a change of the open reading frame. Such modification or inactivation may be accomplished by site-directed mutagenesis or PCR generated mutagenesis in accordance with methods known in the art. Although, in principle, the modification may be performed in vivo, i.e., directly on the cell expressing the nucleic acid sequence to be modified, it is preferred that the modification be performed in vitro as exemplified below.

An example of a convenient way to eliminate or reduce atg11 activity is based on techniques of gene replacement or gene interruption. For example, in the gene interruption method, a nucleic acid sequence corresponding to the endogenous gene or gene fragment of interest is mutagenized in vitro to produce a defective nucleic acid sequence which is then transformed into the host cell to produce a defective gene. By homologous recombination, the defective nucleic acid sequence replaces the endogenous gene or gene fragment. It may be desirable that the defective gene or gene fragment also encodes a marker which may be used for selection of transformants in which the gene encoding the polypeptide has been modified or destroyed. Methods for deleting or disrupting a targeted gene are described, for example, by Miller, et al (1985. Mol. Cell. Biol. 5:1714-1721); WO 90/00192; May, G. (1992. Applied Molecular Genetics of Filamentous Fungi. J. R. Kinghorn and G. Turner, eds., Blackie Academic and Professional, pp. 1-25); and Turner, G. (1994. Vectors for Genetic Manipulation. S. D. Martinelli and J. R. Kinghorn, eds., Elsevier, pp. 641-665).

Alternatively, modification or inactivation of the nucleic acid sequence may be performed by established anti-sense techniques using a nucleotide sequence complementary to the atg11 polypeptide encoding sequence. Anti-sense technology and its application are described, for example, in U.S. Pat. No. 5,190,931.

The above methods for the modification of the atg11 activity may be applied to modify other components of the selective autophagy pathway

The present invention further relates to a modified filamentous fungal cell of a parent cell which comprises a disruption or deletion of a nucleic acid sequence encoding the atg11 protein or a control sequence thereof, which results in the mutant cell producing less of the atg11 protein than the parent cell. Due to genetic modification, the atg11 activity of the filamentous fungal host cell has been disrupted or deleted, such that the fungal host cell either has no atg11 activity or reduced atg11 activity, such as, for example, that the level of atg11 activity expressed by the host cell is individually reduced more than about 50%, preferably more than about 85%, more than about 90%, more than about 95%, more than 98%, or more than 99%. An effective disruption or deletion of the atg11 activity may be assessed by measuring whether the modified fungal host cell has increased yield of the biological product of interest, e.g., polypeptide of interest.

In one aspect, the invention provides a method of producing a polypeptide of interest in a filamentous fungal host cell of comprising a nucleic acid sequence encoding a heterologous polypeptide of intereset, in which the method comprises introducing into the atg11 deficient host cell a nucleic acid sequence encoding the polypeptide of interest, cultivating the host cell in a suitable growth medium, followed by recovery of the protein product. The host cell contains structural and regulatory genetic regions necessary for the expression of the desired polypeptide of interest. The nature of such structural and regulatory regions greatly depends on the polypeptide and the host cell in question. The genetic design of the host cell of the invention may be accomplished by the person skilled in the art using standard recombinant DNA technology for the transformation or transfection of a host cell (vide, e.g., Sambrook et al., inter alia). Preferably, the host cell is modified by methods known in the art for the introduction of an appropriate cloning vehicle, i.e. a plasmid or a vector, comprising a DNA fragment encoding the desired protein product. The cloning vehicle may be introduced into the host cell either as an autonomously replicating plasmid or integrated into the chromosome. Preferably, the cloning vehicle comprises one or more structural regions operably linked to one or more appropriate regulatory regions.

In embodiments of the invention, the filamentous fungal host cell comprising an inactivated (partially or completely) selective autophagy pathway, e.g., by disrupting or deleting the atg11 activity, may further be a protease deficient or protease minus strain in which the expression of one or more endogenous proteases has been reduced or completely elimianted. In a particular embodiment, the parent strain is the protease deficient *Aspergillus oryzae* strain BECh2 described in WO 00/39322, example 1, which is further referring to JaL228 described in WO 98/12300, example 1. This strain is deficient in the alkaline protease Alp and the neutral metalloprotease Npl. Other modifications can include the disruption or deletion of serine protease of the subtilisin type designated PepC and optionally the calcium dependent, neutral, serine protease, KexB, are deficient.

In an embodiment of the invention, it may also be desirable to make other modifications to the fungal host cell to increase expression and/or secretion of a biological product (e.g., polypeptide of interest). For example, the host cell may be modified to increase the presence of other proteins in the fungal cell which are beneficial to increase the secretion of a polypeptide of interest. In a particular embodiment of the invention, the filamentous fungal host cell is modified to increase the presence of a UPR modulating protein in the filamentous fungal host cell. In another embodiment, the filamentous fungal host cell is modified to increase the presence of the hac1 UPR modulating protein (including spliced (e.g., hacAi) and unspliced versions) and/or ire1 UPR modulating protein in the filamentous fungal host cell. See., e.g., Schroder, M. et al, IRE1- and HAC1-independent Transcriptional Regulation in the Unfolded Protein Response of Yeast, Molecular Microbiology 49(3):591-606, 2003; Cox et al., "A Novel Mechanism for Regulating Activity of a Transcription Factor that Controls the Unfolded Protein Response," Cell, vol. 87, pp. 391-404, Nov. 1996; Clarke, H. et al, The Unfolded Protein Response Signal Transducer Ire1p Promotes Secretion of Heterologous Proteins in Saccharomyces cereviseae, J. Cell. Bioch. Suppl., No. 19B, p. 209, 1995; Penttila et al., Activation mechanisms of the HAC1-mediated unfolded protein response in filamentous fungi., Mol Microbiol. 47(4):1149-61, 2003; and Archer et al., HacA-Independent Induction of Chaperone-Encoding Gene bipA in Aspergillus niger Strains Overproducing Membrane ProteinsAppl Environ Microbiol. 2006 January; 72(1): 953-955.

The modified filamentous fungal cells according to the invention are particularly useful for the expression of heterologous polypeptides in which a modified filamentous fungal host cell contains a deletion or disruption of atg11 activity and is also transformed with an expression vector comprising a polypeptide of interest. The filamentous fungal host cell comprising the disrupted or deleted atg11 activity and nucleic acid sequence encoding the heterologous polypeptide of interest may be cultured to produce the polypeptide of interest. The culture broth or medium used may be any conventional medium suitable for culturing the host cell of the invention, and formulated according to the principles of the prior art. The medium preferably contains carbon and nitrogen sources as well as other inorganic salts. Suitable media, e.g. minimal or complex media, are available from commercial suppliers, or may be prepared according to published recipes, as in The Catalogue of Strains, published by The American Type Culture Collection. Rockville Md., USA. 1970.

The appropriate pH for fermentation will often be dependent on such factors as the nature of the host cell to be used, the composition of the growth medium, the stability of the polypeptide of interest, and the like. Consequently, although the host cell of the invention may be cultured using any fermentation process performed at any pH, it is advantageous that the pH of the fermentation process is such that acidic and/or neutral protease activities of the host cell are essentially eliminated or at least significantly reduced. Thus, removal of aspartic protease activity as described in WO 90/00192 may also be accomplished by raising the fermentation pH, and does not present any additional advantageous effect on the yield of a desired protein from host cells cultivated in the alkaline pH range.

If the pH of the fermentation process is within the range from 5 to 11, such as from 6 to 10.5, 7 to 10, or 8 to 9.5, the activity of acidic proteases, such as aspartic and serine proteases, and neutral proteases in the pH ranges above 7, will be reduced or blocked.

Examples of enzymes produced under alkaline fermentation conditions include endoglucanases, phytases and protein disulfide isomerases. However, the alkaline pH range will support alkaline protease activity in an unmodified host cell, which, in turn, may potentially result in degradation of the polypeptide product of interest. Consequently, in such cases the inactivation of the gene encoding alkaline protease is especially advantageous. Inactivation of the alkaline protease gene of the invention is also especially advantageous for certain host cells, as the levels of acidic, neutral and alkaline protease activities vary from species to species.

After cultivation, the desired polypeptide of interest may be recovered by conventional methods of protein isolation and purification from a culture broth, or alternatively, the culture broth may be used directly in an application. Well established purification procedures include separating the cells from the medium by centrifugation or filtration, precipitating proteinaceous components of the medium by means of a salt such as ammonium sulphate, and chromatographic methods such as ion exchange chromatography, gel filtration chromatography, affinity chromatography, and the like.

In a particular embodiment, the disruption or deletion of atg11 activity is applied to produce a heterologous enzyme in an *Aspegrillus niger* host cell. This aspect of the invention provides an *Aspergillus niger* host cell comprising a nucleic acid sequence encoding a heterologous polypeptide of interest further comprising a disruption or deletion of the endogenous atg11 protein activity in the *Aspergillus niger* host cell. In another embodiment, the *Aspergillus niger* host cell comprises a disruption or deletion of the atg11 protein having an amino acid sequence shown in SEQ ID NO:2, encoded by a nucleic acid sequence shown in SEQ ID NO:1.

In yet another embodiment, the *Aspergillus niger* host cell comprises a disruption or deletion of an atg11 protein having an amino acid sequence identity which is at least 50% identical to SEQ ID NO:2. In yet another embodiment, the *Aspergillus niger* host cell comprises a disruption or deletion of an atg11 protein having an amino acid sequence identity which is at least 60% identical to SEQ ID NO:2. In yet another embodiment, the *Aspergillus niger* host cell comprises a disruption or deletion of an atg11 protein having an amino acid sequence identity which is at least 70% identical to SEQ ID NO:2. In yet another embodiment, the *Aspergillus niger* host comprises a disruption or deletion of an atg11 protein having an amino acid sequence identity which is at least 80% identical to SEQ ID NO:2. In yet another embodiment, the *Aspergillus niger* host cell comprise a disruption or deletion of an atg11 protein having an amino acid sequence identity which is at least 90% identical to SEQ ID NO:2. In yet another embodiment, the *Aspergillus niger* host cell comprises a disruption or deletion of an atg11 protein having an amino acid sequence identity which is at least 95% identical to SEQ ID NO:2. In yet another embodiment, an *Aspergillus niger* host cell comprises a disruption or deletion of an atg11 protein having an amino acid sequence identity which is at least 98% identical to SEQ ID NO:2. In yet another embodiment, the *Aspergillus niger* host cell comprises a disruption or deletion of an atg11 protein having an amino acid sequence identity which is at least 99% identical to SEQ ID NO:2.

In yet another embodiment, the *Aspergillus niger* host cell comprises a disruption or deletion of an atg11 gene having a sequence identity which is at least 50% identical to SEQ ID NO:1. In yet another embodiment, the *Aspergillus niger* host cell comprises a disruption or deletion of an atg11 gene having a sequence identity which is at least 60% identical to SEQ ID NO:1. In yet another embodiment, the *Aspergillus niger* host cell comprises a disruption or deletion of an atg11 gene having a sequence identity which is at least 70% identical to SEQ ID NO:1. In yet another embodiment, the *Aspergillus niger* host comprises a disruption or deletion of an atg11 gene having a sequence identity which is at least 80% identical to SEQ ID NO:1. In yet another embodiment, the *Aspergillus niger* host cell comprise a disruption or deletion of an atg11 gene having a sequence identity which is at least 90% identical to SEQ ID NO:1. In yet another embodiment, the *Aspergillus niger* host cell comprises a disruption or deletion of an atg11 gene having a sequence identity which is at least 95% identical to SEQ ID NO:1. In yet another embodiment, an *Aspergillus niger* host cell comprises a disruption or deletion of an atg11 gene having a sequence identity which is at least 98% identical to SEQ ID NO:1. In yet another embodiment, the *Aspergillus niger* host cell comprises a disruption or deletion of an atg11 gene having a sequence identity which is at least 99% identical to SEQ ID NO:1.

In a particular embodiment, the disruption or deletion of atg11 activity is applied to produce a heterologous enzyme in an *Aspegrillus oryzae* host cell. This aspect of the invention provides an *Aspergillus oryzae* host cell comprising a nucleic acid sequence encoding a heterologous polypeptide of interest further comprising a disruption or deletion of the endogenous atg11 protein activity in the *Aspergillus oryzae* host cell.

In yet another embodiment, the disruption or deletion of atg11 activity is applied to produce a heterologous enzyme in a *Trichoderma* host cell, such as, e.g., *Trichoderma harzianum, Trichoderma koningii, Trichoderma longibrachiatum, Trichoderma reesei,* or *Trichoderma viride* cell.. This aspect of the invention provides a *Trichoderma* host cell comprising a nucleic acid sequence encoding a heterologous polypeptide of interest further comprising a disruption or deletion of the endogenous atg11 protein activity in the *Trichoderma* host cell.

Particular heterologous polypeptides of interest that may be produced in the modified filamentous fungal cells (such as, an *Aspergillus niger, Aspgerillus oryzae or Trichoderma* host cells) include enzymes, such as, for example, an enzyme selected from the group consisting of a peptidase (carboxypeptidase, aminopepetidase, protease), amylase (e.g., alpha-amylase or glucoamylase), pullulanase carbohydrase, catalase, cellulase, chitinase, cutinase, cyclodextrin glycosyltransferase, deoxyribonuclease, esterase, endo-glucosidase, alpha-galactosidase, beta-galactosidase, alpha-glucosidase, beta-glucosidase, invertase, laccase, lipase, phospholipase, mannosidase, mutanase, oxidase, pectinolytic enzyme, peroxidase, phytase, polyphenoloxidase, ribonuclease, transglutaminase, and xylanase.

In a particular embodiment, the enzyme is an alpha-amyase. In another particular embodiment, the enzyme is a glucoamylase. In another particular embodiment, the enzyme is a phytase.

The disruption or deletion of the atg11 gene increases the yield of the polypeptide of interest compared to an identical host without the disruption or deletion of the atg11 gene in the same amount of time.

The following preparations and examples are given to enable those skilled in the art to more clearly understand and practice the present invention.

**Materials and Methods**

Unless otherwise stated, DNA manipulations and transformations were performed using standard methods of molecular biology as described in Sambrook et al. (1989) Molecular cloning: A laboratory manual, Cold Spring Harbor lab., Cold Spring Harbor, NY; Ausubel, F. M. et al. (eds.) "Current protocols in Molecular Biology", John Wiley and Sons, 1995; Harwood, C. R., and Cutting, S. M. (eds.) "Molecular Biological Methods for Bacillus". John Wiley and Sons, 1990.

Purchased material (E.coli and kits)

*E.coli* DH5-alpha (Toyobo) is used for plasmid construction and amplification. Amplified plasmids are recovered with Qiagen Plasmid Kit (Qiagen). Ligation is done with DNA ligation kit (Takara) or T4 DNA ligase (Boehringer Mannheim). Polymerase Chain Reaction (PCR) is carried out with Expand TM PCR system (Boehringer Mannheim). QIAquickTM Gel Extraction Kit (Qiagen) is used for the purification of PCR fragments and extraction of DNA fragment from agarose gel.

Plasmids

pBluescript II SK- (Stratagene #212206)

The pHUda801 harbouring *A.nidulans* pyrG gene and herpes simplex virus (HSV) thymidine kinase gene (TK) driven by *A.nidulans* glyceraldehyde-3-phosphate dehydrogenase promoter (Pgpd) and *A.nidulans* tryptophane synthase terminator (TtrpC) are described in example 4 in WO2012/160093.
The pPE001 harboring amyloglucosidase (glucoamylase) gene of interest is described in 12191-EP.
The plasmid pRika147 for the vector of expression of the enzyme genes is described in example 9 in WO2012/160093.

Gene

Atg11 (DSM genome database ID; An02g07380)

Microbial strains

An *Aspergillus niger* expression host strain having the phenotype: pyrG- phenotype/ uridine auxotrophy was used.
MSS was composed of 70 g of sucrose, 100 g of soy bean powder, three drops of pluronic antifoam, and deionized water to 1 liter; pH adjusted to 6.0.
MU1 was comprised of 260 g maltodextrin, 3 g $MgSO_4 \cdot 7H_2O$, 6 g $K_2SO_4$, 5 g $KH_2PO_4$, 0.5 ml of AMG metals solution, three drops of pluronic antifoam, and deionized water to 1 liter; pH adjusted to 4.5.
AMG metals solution was composed of 0.3 g citiric acid•H2O, 0.68 g $ZnCl_2$, 0.25 g $CuSO_4 \cdot 5H_2O$, 0.024 g $NiCl_2 \cdot 6H_2O$, 1.39 g $FeSO_4 \cdot 7H_2O$, 1.356 g $MnSO_4 \cdot 5H_2O$ and deionized water to 1 liter.

**Transformation of *Aspergillus niger***

Transformation of the parent *Aspergillus niger* host cell was achieved using the general methods known for transformation in filamentous fungi, as described in the Yelton et al., "Transformation of Aspergillus nidulans by using a trpC plasmid," Proc Natl Acad Sci USA. 1984 Mar;81(5):1470-4, and as follows:

> *Aspergillus niger* host strain was inoculated to 100 ml of YPG medium supplemented with 10 mM uridine in case the host strain is a pyrG deficient mutant, and incubated for 16 hrs at 32°C at 80 rpm. Pellets were collected and washed with 0.6 M KCI, and resuspended 20 ml 0.6 M KCI containing a commercial β-glucanase product (GLUCANEX™, Novozymes A/S, Bagsvaerd, Denmark) at a final concentration of 20 mg per ml. The suspension was incubated at 32 °C at 80 rpm until protoplasts were formed, and then washed twice with STC buffer. The protoplasts were counted with a hematometer and resuspended and adjusted in an 8:2:0.1 solution of STC:STPC:DMSO to a final concentration of $2.5 \times 10^7$ protoplasts/ml. Approximately 4μg of plasmid DNA was added to 100 μl of the protoplast suspension, mixed gently, and incubated on ice for 30 minutes. One ml of SPTC was added and the protoplast suspension was incubated for 20 minutes at 37°C. After the addition of 10 ml of 50°C osmotic top agarose containing osmotic stabilizer, such as 1 M sucrose, and low melting agarose, the mixture was poured onto the minimum medium (e.g. minimal medium described in COVE 1966 supplemented with 3g/L sodium nitrate as nitrogen source) containing osmotic stabilizer and the plates were incubated at 32°C for 5days.

The components of the media are as following;

> YPG medium was composed of 15 g glucose, 4 g yeast extract, 1 g $K_2HPO_4$, 0.5 g $MgSO_4 \cdot 7H_2O$, and deionized water to 1 liter.(pH 5.7)
> COVE trace metals solution was composed of 0.04 g of $NaB_4O_7 \cdot 10H_2O$, 0.4 g of $CuSO_4 \cdot 5H_2O$, 1.2 g of $FeSO_4 \cdot 7H_2O$, 0.7 g of $MnSO_4 \cdot H_2O$, 0.8 g of $Na_2MoO_2 \cdot 2H_20$, 10 g of $ZnSO_4 \cdot 7H_2O$, and deionized water to 1 liter.

50X COVE salts solution was composed of 26 g of KCl, 26 g of MgSO4•7H2O, 76 g of KH2PO4, 50 ml of COVE trace metals solution, and deionized water to 1 liter.
COVE medium was composed of 342.3 g of sucrose, 20 ml of 50X COVE salts solution, 10 ml of 1 M acetamide, 10 ml of 1.5 M CsCl2, 25 g of Noble agar, and deionized water to 1 liter.
COVE-N (tf) was composed of 342.3 g of sucrose, 3 g of NaNO3, 20 ml of COVE salts solution, 30 g of Noble agar, and deionized water to 1 liter.
COVE-N top agarose was composed of 342.3 g of sucrose, 3 g of NaNO3, 20 ml of COVE salts solution, 10 g of low melt agarose, and deionized water to 1 liter.
COVE-N was composed of 30 g of sucrose, 3 g of NaNO3, 20 ml of COVE salts solution, 30 g of Noble agar, and deionized water to 1 liter.
STC buffer was composed of 0.8 M sorbitol, 25 mM Tris pH 8, and 25 mM CaCl2.
STPC buffer was composed of 40% PEG 4000 in STC buffer.
D.J. Cove, "The Introduction and Repression of Nitrate Reductate in the Fungus Aspgergillus Nidualans," Biochimica et Biophysca ACTA, (1966) 51-56.

**PCR amplification**

| | |
|---|---|
| 5x PCR buffer (incl.$MgCl_2$) | 20 µl |
| 2.5mM dNTP mix | 10 µl |
| Forward primer (1000□µM) | 1 µl |
| Reverse primer (100 µM) | 1 µl |
| Expand High Fidelity polymerase (Roche) | 1 µl |
| Template DNA (50-100 ng/ µl) | 1 µl |
| Distilled water to | 100 µl |

**PCR conditions**

| | | |
|---|---|---|
| 94 C | 2 min | 1 cycle |
| 92C | 1 min | 30 cycles |
| 55C | 1min | |
| 72C | 1-2 min | |
| 72C | 7 min | 1 cycle |

**Lab-scale tank cultivation for glucoamylase production**

Fermentation was done as fed-batch fermentation (H. Pedersen, Glucoamylase production in batch, chemostat and fed-batch cultivations by an industrial strain of Aspergillus niger. Appl Microbiol Biotechnol. Mar; 53(3):272-7, 2000). Selected strains were pre-cultured in liquid media then grown mycelia were transferred to the tanks for further cultivation of enzyme production. Cultivation was done at pH 4.5 at 34C for 7 days with the feeding of glucose and ammonium without over-dosing which prevents enzyme production. Culture supernatant after centrifugation was used for enzyme assay

**Southern hybridization**

Mycelia of the selected transformants were harvested from overnight culture in 100 ml YPG medium, rinsed with distilled water, dried and frozen at -80 °C. Ground mycelia were incubated with Proteinase K and RNaseA at 65 °C for 1 hrs. Genome DNA was recovered by phenol/CHCl3 extraction twice followed by EtOH precipitation and resuspended with distilled water. Non-radioactive probes were synthesized using a PCR DIG probe synthesis kit (Roche Applied Science, Indianapolis IN) followed by manufacture's instruction. DIG labeled probes were gel purified using a QIAquickTM Gel Extraction Kit (QIAGEN Inc., Valencia, CA) according to the manufacturer's instructions.

Five micrograms of genome DNA was digested with appropriate restriction enzymes completely for 16 hours (40 μl total volume, 4U enzyme/μl DNA) and run on a 0.8 % agarose gel. The DNA was fragmented in the gel by treating with 0.2 M HCl, denatured (0.5M NaOH, 1.5M NaCl) and neutralized (1M Tris, pH7.5; 1.5M NaCl) for subsequent transfer in 20X SSC to Hybond N+ membrane (Amersham). The DNA was UV cross-linked to the membrane and prehybridized for 1 hour at 42 °C in 20 ml DIG Easy Hyb (Roche Diagnostics Corporation, Mannheim, Germany). The denatured probe was added directly to the DIG Easy Hyb buffer and an overnight hybridization at 42 °C was done. Following the post hybridization washes (twice in 2X SSC, roome temperature, 5 min and twice in 0.1X SSC, 68°C, 15 min. each), chemiluminescent detection using the DIG detection system and CPD-Star (Roche) was done followed by manufacture's protocol. The DIG-labeled DNA Molecular Weight Marker II (Roche) was used for the standard marker.

**Glucoamylase activity**

Glucoamylase activity is measured in AmyloGlucosidase Units (AGU). The AGU is defined as the amount of enzyme, which hydrolyzes 1 micromole maltose per minute under the standard conditions 37°C, pH 4.3, substrate: maltose 23.2 mM, buffer: acetate 0.1 M, reaction time 5 minutes. An autoanalyzer system may be used. Mutarotase is added to the glucose dehydrogenase reagent so that any alpha-D-glucose present is turned into beta-D-glucose. Glucose dehydrogenase reacts specifically with beta-D-glucose in the reaction mentioned above, forming NADH which is determined using a photometer at 340 nm as a measure of the original glucose concentration.

Amyloglycosidase incubation:

| | |
|---|---|
| Substrate: | maltose 23.2 mM |
| Buffer: | acetate 0.1 M |
| pH: | 4.30 ± 0.05 |
| Incubation temperature: | 37°C ± 1 |
| Reaction time: | 5 minutes |
| Enzyme working range: | 0.5-4.0 AGU/mL |

Color reaction:

| | |
|---|---|
| GlucDH: | 430 U/L |
| Mutarotase: | 9 U/L |
| NAD: | 0.21 mM |
| Buffer: | phosphate 0.12 M; 0.15 M NaCl |

| | |
|---|---|
| pH: | 7.60 ± 0.05 |
| Incubation temperature: | 37°C ± 1 |

| | |
|---|---|
| Reaction time: | 5 minutes |
| Wavelength: | 340 nm |

**Example 1**: **Disruption of atg11 gene in *Aspergillus niger.***

Construction of the atg11 gene disruption plasmid pHUda1493

The following primers 3atg11 F and 3atg11 R introducing a XbaI site and a EcoRI site, respectively, were designed to isolate a 3' flanking region at the position between +26800 and +4770 from the atg start codon of the *A.niger* atg11 gene based on the nucleotide sequences information in *Aspergillus niger* genome DNA database An02g07380.

3atg11 F: actagttctagagttgagacattggctcgt (SEQ ID NO:3)
3atg11R: gaattcgctagccgtccagagacctaggg (SEQ ID NO:4)

A PCR reaction with genome DNA of *Aspergillus niger* strain as template was performed using a primer pair of 3atg11 F and 3atg11 R. The reaction products were isolated on a 1.0% agarose gel and 2.0 kb product band was excised from the gel. The 2.0 kb amplified DNA fragment was digested with EcoRI and Xbal, and ligated into the pHUda801 digested with EcoRI and Xbal to create pHUda1492.

The following primers 5atg11 F and 5atg11 R introducing a Notl site and a Spel site, respectively, were designed to isolate a 5' flanking region at the position between -1970 and -10 from the atg start codon of *A.niger* atg11 gene based on the nucleotide sequences information in *Aspergillus niger* genome DNA database An02g07380.

5atg11 F: gcggccgcggtcggcgttcgaagg (SEQ ID NO:5)
5atg11 R: tactagtgggggtatcgaagtcttcggggc (SEQ ID NO:6)

A PCR reaction with genome DNA of *Aspergillus niger* strain as template was performed using a primer pair of 5atg11 F and 5atg11 R. The reaction products were isolated on a 1.0% agarose gel and 2.0 kb product band was excised from the gel. The 2.0 kb amplified DNA fragment was digested with Notl and Spel, and ligated into the pHUda1492 digested with Notl and Spel to create pHUda1493.

The atg11 gene disruption in Aspergillus niger parent strain

The pHUda1493 was introduced into the *Aspergillus niger* parent strain using a standard method for A. niger transformation as described in Yelton et al., "Transformation of Aspergillus nidulans by using a trpC plasmid," Proc Natl Acad Sci USA. 1984 Mar;81(5):1470-4). Randomly selected transformants were inoculated onto the minimum medium plates with 2.5 μM 5-Flouro-2-deoxyuridine (FdU), an agent which kills cells expressing the herpes simplex virus (HSV) thymidine kinase gene (TK) harbouring in pHUda1493. Strains which grew well on such a plate with 2.5 μM FdU were purified and subjected to Southern blotting analysis to confirm whether the atg11 gene was disrupted correctly or not.

The following set of primers to make non-radioactive probe was used to analyze the selected transformants described below. It generates the probe for 3' atg11 flanking region;, forward primer: tggctcgtcagtctgaaga, reverse primer: tacacgcttgacgaccgcgt.

Genomic DNA extracted from the selected transformants was digested by Spel.
By the right gene disruption event, a hybridized signal at the size of 12.7 kb by Spel digestion was shifted to 5.9 kb probed described above. Among the strains given the right integration events, a strain Huda1493 was selected.

**Example 2: Production of atg11 deficient recombinant host cell containing a gene of interest**

Construction of glucoamylase (AMG) expression plasmid pHUda1479

Primers PE001-F and PE001-R introduced a *Bam* HI site and a *Pml* I site, respectively, and were designed to isolate the glucoamylase variant gene.
PE001-F:

cggatccaccatgcgtctcactctattatc (SEQ ID NO: 7)

PE001-R:

accacgtgtcaaaactgccacacgtcgt (SEQ ID NO: 8)

A PCR reaction with plasmid pPE001 as template was performed using a primer pair of PE0011 F and PE001 R. The reaction products were isolated on a 1.0% agarose gel and 1.9 kb product band was excised from the gel. The 1.9 kb amplified DNA fragment was digested with BamHI and Pmll, and ligated into the pRika147 digested with BamHI and Pmll to create pHUda1479.

Construction of the Aspergillus niger transcription factor hacA expression plasmid expression plasmid pHUda1292

Based on the sequence information on the transcription factor hacA from literature (The transcription factor hacA mediates the unfolded protein response in Aspergillus niger, and up-regulates its own transcription. Mulder HJ, Saloheimo M, Penttilä M, Madrid SM. Mol Genet Genomics. 2004 Mar;271(2):130-40. Epub 2004 Jan 17.), the following primers were made.

hacA1: tttgctagccgacggggatgctttttgc (SEQ ID NO: 9)
hacA2: cttccatcatggtggatccttcaagcgtgacag (SEQ ID NO: 10)
hacA3: ctgtcacgcttgaaggatccaccatgatggaag (SEQ ID NO: 11)
hacA4; ccagcgacggacactgcaggatgttgtgtc (SEQ ID NO: 12)
hacA5: gacacaacatcctgcagtgtccgtcgctgg (SEQ ID NO: 13)
hacA6; ttcacgtgataaaattataaggatt (SEQ ID NO: 14)

A PCR reaction with genome DNA of *Aspergillus niger* as template was performed using a primer pair of hacA1 & 2, hacA 3 &4 and hacA 5 & 6. The reaction products were isolated on a 1.0% agarose gel and 1.3, 0.7 and 0.7 kb product band was excised from the gel. These three fragments were mixed and used for the 2nd PCR reaction using a primer pair of hacA 1 & 6. The reaction products were isolated on a 1.0% agarose gel and 2.7 kb product band was excised from the gel. The 2.7 kb amplified DNA fragment was digested with NheI and PmlI, and ligated into the pRika147 digested with NheI and PmlI. to create pHUda1292.

The pyrG gene rescue in huda1493

The introduced pyrG gene at the atg11 loci in huda1493 was rescued as follows. The strain huda1493 was inoculated once on the minimum medium containing 10 mM uridine and 1 g/ L 5-fluoro-orotic acid (5-FOA). Strains in which the *pyrG* gene has been deleted will grow in the presence of 5-FOA; those that retain the gene will convert 5-FOA to 5-fluoro-UMP, a toxic intermediate. The colonies that grew fast were isolated. The isolates strain was named huda1493-3.

Co-expression of AMG and hacA in the atg11 gene disrupted strain huda1493-3

Competitive gene swapping using pHUda1292 and pHUda1479 to create strains having altered gene copies of the AMG and hacA was performed.

The pHUda1292 and phuda1479 were co-introduced into Aspergillus niger strain huda1493-3 and its parent strain. Transformants were selected from the standard medium supplemented with 1% D-xylose and 10 μg/ml 5-fluorocytosine (5FC). Randomly selected transformants were inoculated onto the minimum medium plates supplemented with 10 μg/ml 5-fluorocytosine (5FC). Strains which grew well were purified and subjected to Southern blotting analysis to confirm whether the AMG gene in phuda1479 and the hacA gene in pHUda1292 was introduced at NA1, NA2, SP288 or PAY loci correctly or not.

The following set of primers to make non-radioactive probe was used to analyze the selected transformants.

For AMG coding region:

forward primer: ttatacatggactcgtgact (SEQ ID NO: 15)
reverse primer: agaatcacaggcatcgcagg (SEQ ID NO: 16)

For hacA coding region;

forward primer: agaagagaaagtcatggggc (SEQ ID NO: 17)
reverse primer: agggcacttccttctccttc (SEQ ID NO: 18)

Genomic DNA extracted from the selected transformants was digested by SpeI and PmlI, then probed with AMG coding region. By the right gene introduction event, hybridized signals at the size of 6.9 kb (NA1), 3.6 kb (SP288), 4.9 kb (NA2) and 5.8 kb (PAY) by SpeI and PmlI digestion was observed probed described above.

Genomic DNA extracted from the selected transformants was digested by SphI and probed with hacA coding region. By the right gene introduction event, hybridized signals at the size of 8.6 kb (NA1), 4.2 kb (SP288), 5.3 kb (NA2), 3.9 kb (PAY) and 2.2 kb (native gene) by SphI digestion was observed probed described above.

The three transformants having the AMG gene in the pHUda1479 at NA1 and SP288 loci and hacA gene in the pHUda1292 at the NA2 and PAY loci were selected from both huda1493-3 and the parent.

**Example 3: Effect of the atg11 gene disruption on enzyme production.**

Three strains from the parent and the other three strains from huda1493-3 introducing the AMG gene at 2 loci and the additional hacA gene at 2 loci were fermented in lab-scale tanks and their enzyme activities (AGU activities) were measured followed by the methods described above; results are shown in the table below.

Table. The average AGU activity of the selected three strains from each host strain, wherein the average AMG yields from the parent is normalized to 1.00.

| host strain | AMG copies | AGU relative activity |
|---|---|---|
| Parent strain | 2 | 1.00 |
| huda1493-3 | 2 | 1.30 |

## Claims

1. A filamentous fungal cell which comprises a complete or partial inactivation of the atg11 activity of said filamentous fungal cell, wherein the cell comprises an expression vector which comprises a sequence of nucleotides that encodes a heterologous enzyme of interest, and genetic elements suitable for producing the heterologous enzyme in the filamentous fungal cell.

2. The filamentous cell fungal cell of claim 1, wherein the heterologous enzyme of interest is selected from the group consisting of a peptidase (carboxypeptidase, aminopepetidase, protease), amylase (e.g., alpha-amylase or glucoamylase), carbohydrase, pullulanase, catalase, cellulase, chitinase, cutinase, cyclodextrin glycosyltransferase, deoxyribonuclease, esterase, endo-glucosidase, alpha-galactosidase, beta-galactosidase, alpha-glucosidase, beta-glucosidase, invertase, laccase, lipase, phospholipase, mannosidase, mutanase, oxidase, pectinolytic enzyme, peroxidase, phytase, polyphenoloxidase, ribonuclease, transglutaminase, and xylanase.

**3.** The filamentous fungal cell of claim 1, wherein the heterologous enzyme of interest is a glucoamylase.

**4.** The filamentous fungal cell of claim 1, wherein the heterologous enzyme of interest is an amylase.

**5.** The filamentous fungal cell of any of the preceding claims, wherein the filamentous fungal cell is selected from the group consisting of *Acremonium, Aspergillus, Fusarium, Humicola, Mucor, Myceliophthora, Neurospora, Penicillium, Thielavia, Tolypocladium,* and *Trichoderma*.

**6.** The filamentous fungal cell of any of the preceding claims, wherein the filamentous fungal cell is selected from the group consisting of *Aspergillus awamori, Aspergillus foetidus, Aspergillus japonicus, Aspergillus nidulans, Aspergillus niger, Aspergillus oryzae, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Thielavia terrestris, Trichoderma harzianum, Trichoderma koningii, Trichoderma longibrachiatum, Trichoderma reesei,* and *Trichoderma viride* cell.

**7.** The filamentous fungal cell of any of the preceding claims, wherein the filamentous fungal cell is an *Aspergillus* cell.

**8.** The filamentous fungal cell of any of the preceding claims, wherein the filamentous fungal cell is an *Aspergillus niger* or *Aspergillus oryzae* cell.

**9.** The filamentous fungal cell of any of the preceding claims, which comprises a deletion or disruption of upstream or downstream regulatory sequences of the atg11 gene.

**10.** The filamentous fungal cell of any of claims 1-9, which comprises a deletion or disruption of the atg11 gene.

**11.** The filamentous fungal cell of any of the preceding claims, which comprises a deletion of the atg11 gene.

**12.** The filamentous fungal cell of any of the preceding claims wherein the fungal cell further comprises a modification to increase the presence of an hac1 UPR modulating protein in the filamentous fungal host cell.

**13.** The filamentous fungal cell of any of the preceding claims wherein the fungal cell further comprises a modification to increase the presence of an ire1 UPR modulating protein in the filamentous fungal host cell.

**14.** A method for producing a heterologous polypeptide of interest comprising culturing a filamentous fungal cell of any of the preceding claims under conditions conducive to induce expression of a heterologous polypeptide of interest.

**15.** The method of claim 14, further comprising recovering the polypeptide of interest from the host cell culture medium.

## Patentansprüche

**1.** Zelle eines filamentösen Pilzes, die eine vollständige oder teilweise Inaktivierung der atg11-Aktivität der Zelle eines filamentösen Pilzes umfasst, wobei die Zelle einen Expressionsvektor, der eine Sequenz von Nukleotiden umfasst, die ein heterologes Enzym von Interesse kodieren, und genetische Elemente, die zum Herstellen des heterologen Enzyms in der Zelle eines filamentösen Pilzes geeignet sind, umfasst.

**2.** Zelle eines filamentösen Pilzes nach Anspruch 1, wobei das heterologe Enzym von Interesse ausgewählt ist aus der Gruppe bestehend aus einer Peptidase (Carboxypeptidase, Aminopeptidase, Protease), Amylase (z.B. alpha-Amylase oder Glucoamylase), Carbohydrase, Pullulanase, Katalase, Cellulase, Chitinase, Cutinase, Cyclodextringlycosyltransferase, Desoxyribonuklease, Esterase, endo-Glucosidase, alpha-Galactosidase, beta-Galactosidase, alpha-Glucosidase, beta-Glucosidase, Invertase, Laccase, Lipase, Phospholipase, Mannosidase, Mutanase, Oxidase, pektinolytischem Enzym, Peroxidase, Phytase, Polyphenoloxidase, Ribonuklease, Transglutaminase und Xylanase.

3. Zelle eines filamentösen Pilzes nach Anspruch 1, wobei das heterologe Enzym von Interesse eine Glucoamylase ist.

4. Zelle eines filamentösen Pilzes nach Anspruch 1, wobei das heterologe Enzym von Interesse eine Amylase ist.

5. Zelle eines filamentösen Pilzes nach einem beliebigen der voranstehenden Ansprüche, wobei die Zelle eines filamentösen Pilzes ausgewählt ist aus der Gruppe bestehend aus *Acremonium, Aspergillus, Fusarium, Humicola, Mucor, Myceliophthora, Neurospora, Penicillium, Thielavia, Tolypocladium* und *Trichoderma.*

6. Zelle eines filamentösen Pilzes nach einem beliebigen der voranstehenden Ansprüche, wobei die Zelle eines filamentösen Pilzes ausgewählt ist aus der Gruppe bestehend aus einer Zelle von *Aspergillus awamori, Aspergillus foetidus, Aspergillus japonicus, Aspergillus nidulans, Aspergillus niger, Aspergillus oryzae, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Thielavia terrestris, Trichoderma harzianum, Trichoderma koningii, Trichoderma longibrachiatum, Trichoderma reesei* und *Trichoderma viride*.

7. Zelle eines filamentösen Pilzes nach einem beliebigen der voranstehenden Ansprüche, wobei die Zelle eines filamentösen Pilzes eine *Aspergillus*-Zelle ist.

8. Zelle eines filamentösen Pilzes nach einem beliebigen der voranstehenden Ansprüche, wobei die Zelle eines filamentösen Pilzes eine *Aspergillus niger*- oder *Aspergillus oryzae*-Zelle ist.

9. Zelle eines filamentösen Pilzes nach einem beliebigen der voranstehenden Ansprüche, die eine Deletion oder Unterbrechung von stromaufwärts oder stromabwärts gelegenen regulatorischen Sequenzen des atg11-Gens umfasst.

10. Zelle eines filamentösen Pilzes nach einem beliebigen der Ansprüche 1-9, die eine Deletion oder Unterbrechung des atg11-Gens umfasst.

11. Zelle eines filamentösen Pilzes nach einem beliebigen der voranstehenden Ansprüche, die eine Deletion des atg11-Gens umfasst.

12. Zelle eines filamentösen Pilzes nach einem beliebigen der voranstehenden Ansprüche, wobei die Pilzzelle weiterhin eine Modifikation umfasst, um die Gegenwart eines hac1 UPR-modulierenden Proteins in der Wirtszelle eines filamentösen Pilzes zu erhöhen.

13. Zelle eines filamentösen Pilzes nach einem beliebigen der voranstehenden Ansprüche, wobei die Pilzzelle weiterhin eine Modifikation umfasst, um die Gegenwart eines ire1 UPR-modulierenden Proteins in der Wirtszelle eines filamentösen Pilzes zu erhöhen.

14. Verfahren zum Herstellen eines heterologen Polypeptids von Interesse, umfassend Kultivieren einer Zelle eines filamentösen Pilzes gemäß einem beliebigen der voranstehenden Ansprüche unter Bedingungen, die zum Induzieren von Expression eines heterologen Polypeptids von Interesse förderlich sind.

15. Verfahren nach Anspruch 14, weiterhin umfassend das Gewinnen des Polypeptids von Interesse aus dem Wirtszellkulturmedium.

**Revendications**

1. Cellule fongique filamenteuse qui comporte une inactivation complète ou partielle de l’activité de l’atg11 de ladite cellule fongique filamenteuse, dans laquelle la cellule comprend un vecteur d’expression qui comprend une séquence de nucléotides qui code pour une enzyme hétérologue d’intérêt, et les éléments génétiques convenables pour la production de l’enzyme hétérologue dans la cellule fongique filamenteuse.

2. Cellule fongique filamenteuse selon la revendication 1, dans laquelle l'enzyme hétérologue d'intérêt est choisie dans le groupe constitué par une peptidase (carboxypeptidase, aminopeptidase, protéase), une amylase (p. ex., alpha-amylase ou glucoamylase), une carbohydrase, une pullulanase, une catalase, une cellulase, une chitinase, une cutinase, une cyclodextrine glycosyltransférase, une désoxyribonucléase, une estérase, une endo-glucosidase, une alpha-galactosidase, une bêta-galactosidase, une alpha-glucosidase, une bêta-glucosidase, une invertase, une laccase, une lipase, une phospholipase, une mannosidase, une mutanase, une oxydase, une enzyme pectinolytique, une peroxydase, une phytase, une polyphénoloxydase, une ribonucléase, une transglutaminase, et une xylanase.

3. Cellule fongique filamenteuse selon la revendication 1, dans laquelle l'enzyme hétérologue d'intérêt est une glucoamylase.

4. Cellule fongique filamenteuse selon la revendication 1, dans laquelle l'enzyme hétérologue d'intérêt est une amylase.

5. Cellule fongique filamenteuse selon l'une quelconque des revendications précédentes, dans laquelle la cellule fongique filamenteuse est choisie dans le groupe constitué par *Acremonium, Aspergillus, Fusarium, Humicola, Mucor, Myceliophtora, Neurospora, Penicillium, Thielavia, Tolypocladium,* et *Trichoderma*.

6. Cellule fongique filamenteuse selon l'une quelconque des revendications précédentes, dans laquelle la cellule fongique filamenteuse est choisie dans le groupe constitué par une cellule d'*Aspergillus awamori, Aspergillus foetidus, Aspergillus japonicus, Aspergillus nidulans, Aspergillus niger, Aspergillus oryzae, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophtora thermophila, Neurospora crassa, Penicillium purpurogenum, Thielavia terrestris, Trichoderma harzianum, Trichoderma koningii, Trichoderma longibrachiatum, Trichoderma reesei,* et *Trichoderma viride*.

7. Cellule fongique filamenteuse selon l'une quelconque des revendications précédentes, dans laquelle la cellule fongique filamenteuse est une cellule *Aspergillus.*

8. Cellule fongique filamenteuse selon l'une quelconque des revendications précédentes, dans laquelle la cellule fongique filamenteuse est une cellule *Aspergillus niger* ou d'*Aspergillus oryzae*.

9. Cellule fongique filamenteuse selon l'une quelconque des revendications précédentes, qui comprend une délétion ou une interruption des séquences de régulation amont ou aval du gène atg11.

10. Cellule fongique filamenteuse selon l'une quelconque des revendications 1 à 9, qui comprend une délétion ou une interruption du gène atg11.

11. Cellule fongique filamenteuse selon l'une quelconque des revendications précédentes, qui comprend une délétion du gène atg11.

12. Cellule fongique filamenteuse selon l'une quelconque des revendications précédentes dans laquelle la cellule fongique comporte en outre une modification destinée à accroître la présence d'une protéine de modulation UPR hac1 dans la cellule hôte fongique filamenteuse.

13. Cellule fongique filamenteuse selon l'une quelconque des revendications précédentes dans laquelle la cellule fongique comprend en outre une modification destinée à accroître la présence d'une protéine de modulation UPR ire1 dans la cellule hôte fongique filamenteuse.

14. Méthode de production d'un polypeptide hétérologue d'intérêt comprenant la culture d'une cellule fongique filamenteuse selon l'une quelconque des revendications précédentes dans des conditions aptes à induire l'expression d'un polypeptide hétérologue d'intérêt.

15. Méthode selon la revendication 14, comprenant en outre la récupération du polypeptide d'intérêt à partir du milieu de culture de la cellule hôte.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9600787 A
- WO 9533836 A
- EP 238023 A
- US 4399216 A
- WO 9000192 A
- US 5190931 A
- WO 0039322 A
- WO 9812300 A
- WO 2012160093 A


### Non-patent literature cited in the description


- **MARTEN et al.** Autophagy in filamentous fungi. *Fungal Genetics and Biology,* 2009, vol. 46, 1-8
- **ASKEW et al.** Unexpected link between Metal Ion Deficiency and Autophagy in Aspergillus fumigatus. *Eukaryotic Cell,* December 2007, 2437-2447
- **KITAMOTO et al.** Functional anaylysis of the ATG8 Homologoue Aoatg8 and Role of Autophagy in Differentiation and Germination of Aspergillus oryzae. *Eukaryotic Cell,* August 2006, 1328-1336
- **KITAMOTO K. ; KIKUMA T.** Analysis of autophagy in Aspergillus oryzae by disruption of Aoatg13, Aoatg4, and Aoatg15 genes. *FEMS Microbiol Lett,* 2011, vol. 316, 61-69
- **KLIONSKY et al.** The Atg1 Kinase Complex Is Involved in the Regulation of Protein Recruitment to Initiate Sequestering Vesicle Formation for Nonspecific Autophagy in Saccharomyces cerevisiaes. *Molecular Biology of the Cell,* 19 February 2008, 668-681
- **VAN DER KLEI.** Autophagy Deficiency Promotes β-Lactam Production in Penicilium chrysogenum. *Applied and Environmental Microbiology,* February 2011, vol. 77 (4), 1413-1422
- **KLIONSKY et al.** The Actin Cytoskeleton Is Required For Selective Types of Autophagy, but Not Nonspecific Autophagy, in the Yeast Saccharomyces cerevisiae. *Molecular Biology of the Cell,* December 2005, (16), 5843-5865
- **KLIONSKY D. J. ; CHEONG H.** Dual role of Atg1 in regulation of autophagy-specific PAS assembly in Saccharomyces cerevisiae. *Autophagy,* July 2008, vol. 4 (5), 724-726
- **CHUNG et al.** ATG1, and autophagy regulator, inhibit cell growth by negatively regulating S6 kinase. *EMBO reports,* 2007, vol. 8 (4), 360-365
- **MIZUSHIMA.** The role of the atg1/ULK1 complex in autophagy regulation. *Current Opinion in Cell Biology,* 2010, vol. 22, 132-139
- **KITAMOTO et al.** *Functional analysis of Aoatg1 and detection of the Cvt pathway in Aspergillus oryzae,* 2012
- **KLIONSKY D. ; YORIMITSU T.** Atg11 Links Cargo to the Vesicle-forming Machinery in the Cytoplasm to Vaculoe Targeting Pathway. *Molecular Biology of the Cell,* April 2005, vol. 16, 159301605
- **K. SUZUKI.** Selective autophagy in budding yeast. *Cell Death and Differentiation,* 2013, vol. 20, 43-48
- **MCKNIGHT G. L. ; O'HARA P. J. ; PARKER M. L.** Nucleotide sequence of the triosephosphate isomerase gene from Aspergillus nidulans: Implications for a differential loss of introns. *Cell,* 1986, vol. 46, 143-147
- **INNIS et al.** PCR Protocols: A Guide to Methods and Application. Academic Press, 1990
- **YELTON et al.** *Proceedings of the National Academy of Sciences USA,* 1984, vol. 81, 1470-1474
- **MALARDIER et al.** *Gene,* 1989, vol. 78, 147-156
- **VAN SOLINGEN et al.** *J. Bact.,* 1977, vol. 130, 946
- **HSIAO et al.** *Proc. Natl. Acad. Sci.,* 1979, vol. 76, 3829
- **KEOWN et al.** *Methods in Enzymology,* 1990, vol. 185, 527-537
- **MANSOUR et al.** *Nature,* 1988, vol. 336, 348-352
- **NEEDLEMAN ; WUNSCH.** *J. Mol. Biol.,* 1970, vol. 48, 443-453
- **RICE et al.** *Trends Genet.,* 2000, vol. 16, 276-277
- **MILLER et al.** *Mol. Cell. Biol.,* 1985, vol. 5, 1714-1721
- **MAY, G.** Applied Molecular Genetics of Filamentous Fungi. Blackie Academic and Professional, 1992, 1-25
- **TURNER, G.** Vectors for Genetic Manipulation. Elsevier, 1994, 641-665

- **SCHRODER, M. et al.** IRE1- and HAC1-independent Transcriptional Regulation in the Unfolded Protein Response of Yeast. *Molecular Microbiology,* 2003, vol. 49 (3), 591-606
- **COX et al.** A Novel Mechanism for Regulating Activity of a Transcription Factor that Controls the Unfolded Protein Response. *Cell,* November 1996, vol. 87, 391-404
- **CLARKE, H. et al.** The Unfolded Protein Response Signal Transducer Ire1p Promotes Secretion of Heterologous Proteins in Saccharomyces cereviseae. *J. Cell. Bioch. Suppl.,* 1995, 209
- **PENTTILA et al.** Activation mechanisms of the HAC1-mediated unfolded protein response in filamentous fungi. *Mol Microbiol.,* 2003, vol. 47 (4), 1149-61
- **ARCHER et al.** HacA-Independent Induction of Chaperone-Encoding Gene bipA in Aspergillus niger Strains Overproducing Membrane. *ProteinsAppl Environ Microbiol.,* January 2006, vol. 72 (1), 953-955
- **SAMBROOK et al.** Molecular cloning: A laboratory manual. Cold Spring Harbor lab, 1989
- Current protocols in Molecular Biology. John Wiley and Sons, 1995
- Molecular Biological Methods for Bacillus. John Wiley and Sons, 1990
- **YELTON et al.** Transformation of Aspergillus nidulans by using a trpC plasmid. *Proc Natl Acad Sci USA,* March 1984, vol. 81 (5), 1470-4
- **H. PEDERSEN.** Glucoamylase production in batch, chemostat and fed-batch cultivations by an industrial strain of Aspergillus niger. *Appl Microbiol Biotechnol.,* March 2000, vol. 53 (3), 272-7
- **YELTON et al.** Transformation of Aspergillus nidulans by using a trpC plasmid. *Proc Natl Acad Sci USA.,* March 1984, vol. 81 (5), 1470-4
- **MULDER HJ ; SALOHEIMO M ; PENTTILÄ M ; MADRID SM.** *Mol Genet Genomics,* March 2004, vol. 271 (2), 130-40

SEQUENCE LISTING

<110> Novozymes A/S Udagawa, Hiroaki

<120> MODIFIED FUNGAL HOST CELLS AND METHOD OF USING SAME

<130> 12580-WO-PCT

<160> 18

<170> PatentIn version 3.5

<210> 1
<211> 3909
<212> DNA
<213> Aspergillus niger

<400> 1

atgactgctc gtgggaaaaa cgttaagacg cttgccacgg agagcgaaat ctttgtctat 60
gaccgaagat gggtcagcga gcccaacagc atccagattc ccgagctacc cccgccacat 120
ccactgcacg tcgataaccc gcccgatacc ctcacaaacc agaacgatct acaagcatgg 180
aggagcctct acttagcaag gaggacatgg gccgcggacc tcacggagcg ctgcgagtcg 240
gcggaaaagc agatacatga gcacaacgag cgcactgata tcatcaaccg agctgcaagc 300
gtagctctcg agaacctcaa aacccacgtc ggcaccctag agcataaatt ccaagaggcc 360
cagtccttcg ccaatgaact tttaagtcaa cagcaagcag cactcaatgg atggaaaagg 420
gcattgtcaa ctttagatag cattccagct cgcaaggact tccccttcct gggccgtccg 480
tcaacaccaa cgaaaggcag aggccaagcg atgggtacac tacaagacta cgtggataca 540
gcggaggtac aaagggctgg ctcggaagcc ccagaaatac ttgcgcgctt tgcccatcaa 600
gttagcacta tcgagaaaaa tgttagtgag attgctacag atactcaacg cctggttgat 660
gaagcctttc ctacgagtgt tgagcccgtg gatggactct tgcaggaaat ggaaacaata 720
tcaaataaga tacaatccga ctacgagcat gttctggcct tgcctaacaa cccaaagaca 780
ttggcaaacg tttctaggct cgctctcagc catacacaag accttctacc atcgttattg 840
gacgttagca acgaaataca ggctggccag gaagaagctg tgagacgata taatgcggca 900
gtgaaagcct cgtcgagtca tatgcggctc atatcttcta ttgagtatcg gcttgccgaa 960
actctacctc agatcaacag cctgcctttg caaagcgaag catttgatgt catttacacg 1020
gtcttccata tgcctattat ctacgggtcg gttctgattg agtctgtgcg acgtcgcgag 1080
ttcaatgata aaatgaagaa cgactcactg actttggcgg aggaaatgtc tgtattccgg 1140
gaagaagagc agcgtcgaag gaagaaatgg ctcaaaaata tggctgattt tatttccatc 1200
tctgatgcta cgacccctgg catcgagata aatctacggg gccaggagca ggaatggcct 1260
gccgttagta ggaaagaagt tgagtcatat atcgaagacc tgaaaaataa acctgacatg 1320

gaaaatcacg cccaagaact gatgcagcgc ttcaaagagc ttgatgctcc tacccggatg 1380
cagcgtcgca gagcaaaagc tttcaaacag ggaagtcttt ttgacctgag ccgaagctcg 1440
cttttactgc gcagtgacga tatggtcaga agtctaaaag atgagaaagt caagcttgaa 1500
gatagattga aaggctccga gagccgtatt cgaaagttag aggatctcct tcatcgtcag 1560
agtcatatga gccgaccctc cagcggcaac ttctcacttg atttccccag ttcaccggcg 1620
tcgcctcacc ccgactcact ttcaaggaga tcttcggtct cttcaaggcg aatgtctgca 1680
aatcaagctc cggaggaaaa ggcgctcgta caacgtatca ctcgcctcga ggccgagcta 1740
gcggctgagc gagacacggt gcaacgactt caaaaggagg cacatgccga gcggcagtct 1800
aataccgaca aaatacaaga agcccagtca acgaagaaag atctcattgg caatctcgaa 1860
gcgcggcaac gtgagtttga tgacgagcgc agatttttgg agggtgaatt aagaaggctt 1920
cgaatccgga ctgaggagct agaagaggag gtggacagaa atctcgacag tcgcgaacac 1980
gagaagcaag acgccgatga gcgcatacat catctcgaga tggaattgca agatgtccat 2040
gcccgagctg aagaagaatt gaggaaagca aatgaaacga ttggacactt gcaatcggag 2100
aagggcgtag gagaaaccct acgggcccgt attgaagagc tacagaacca ggtggctgaa 2160
aacgaaagga aagaaaacga taacaagcac gctttgcagg tcgcttttct caatttgtct 2220
cctggtggct ctgttcctac cgaatttccg aatatcatta aagcaattga ggttctctcc 2280
gaagggctaa caatccatgc caagaatgcg gaggagagtg ccgcaaaagc ttcagcggaa 2340
agcaaggtgc tcgaagaacg gttgggtctg atggagtctg aattggagga tttgagaaag 2400
actgcgacct cacgagaaac agaactttcc caagctcgaa atgaattggc tgaaggaaaa 2460
gcaaagctga gcattgtgac ctcagaattg gaagatgaac gatcaaaact catcgcatta 2520
cagtcccaat ttgctgcggg cgagacagga tccgacgccc tgcgggagcg tgttaccgaa 2580
gaagaacgga agttaactga tctggcccag aggctagctg aggttgagac attggctcgt 2640
cagtctgaag aagaagttac tacctggaag aagaaggttg aagcaatgtc cgaagcggag 2700
cagcaaacta ctgtacggtt cgagacttgc ggggtcaggt tccaggagtt gtcaaagcag 2760
cttttcgcac aagtggagaa gctggagcgc atgctggaac aactagggtt cacgatcgtt 2820
cgtcaaaatg gtgacatagt tgtgcagagg tcgtccaagg tcaatgccct ctctagcacc 2880
ggtgacagtc ttagccagtc gggagtggtg tctgtgagac cggacgcaag ccttctcgat 2940
tggatgcatg ccgatagcaa ggaagaagaa acagataagt tcatggcttt cgtggaatcc 3000
ttatatcaat ttgatgtgga catcttcggc gacgcggtcg tcaagcgtgt aaaggatatt 3060
gaagttcttg ctcgcaaatg gcagaaagaa gcccgcggat accgagacaa gtaccaccgg 3120
gttcaaagcg aagcccatga taaaatcgcc taccggtcct ttaaagaagg ggatcttgca 3180
ctcttcttgc ctacccgcaa ccaagctatc cggtcatggg ctgcgttcaa tgttggcgca 3240

ccacactatt tcctgcgcga gcaagatgcg cacaagcttc aaacccggga ctggctgctt 3300

gctcggatta ccaagatcga agaacgcgtt gttgatctct ccaaatcgat gaacggtgcc 3360

aatcctgacc gccgttccat cggcgaagtc agcgatggca cctcacttga tgacgaaaac 3420

ccgtttgagc tgtctgatgg tctccgctgg tacctcttag atgccacgga agaaaagcca 3480

ggcgccccag cgactccggg cttaggaaag agcactgttg cgccagcaca cgtagacgcc 3540

aaaggcagca ttcgcatgaa acgcacatca gccggtggga atattgccaa aacgttgacc 3600

aagagcttag acagccgacg aaatagttcc aattctaaga aggggcattc ctcgactcct 3660

tcacaacgcg gcaacgaatc aacaacagac ctagcaagac tagctgaaca agacactgct 3720

gccagcttac actcacggga ggcagccccc aaacccgatg agattttaga gatctttgcg 3780

gttggaagaa cagagtataa agctagttta caggttgaac ctaaattaga tgccctttgg 3840

aacgaaagat ctcctgctcc tgaatgccgg gcaaacgagg cagtcaatta cgctaagaaa 3900

ggagagtga 3909

<210> 2
<211> 1302
<212> PRT
<213> Aspergillus niger

<400> 2

```
Met Thr Ala Arg Gly Lys Asn Val Lys Thr Leu Ala Thr Glu Ser Glu
1               5                   10                  15

Ile Phe Val Tyr Asp Arg Arg Trp Val Ser Glu Pro Asn Ser Ile Gln
            20                  25                  30

Ile Pro Glu Leu Pro Pro Pro His Pro Leu His Val Asp Asn Pro Pro
        35                  40                  45

Asp Thr Leu Thr Asn Gln Asn Asp Leu Gln Ala Trp Arg Ser Leu Tyr
    50                  55                  60

Leu Ala Arg Arg Thr Trp Ala Ala Asp Leu Thr Glu Arg Cys Glu Ser
65                  70                  75                  80

Ala Glu Lys Gln Ile His Glu His Asn Glu Arg Thr Asp Ile Ile Asn
                85                  90                  95

Arg Ala Ala Ser Val Ala Leu Glu Asn Leu Lys Thr His Val Gly Thr
            100                 105                 110

Leu Glu His Lys Phe Gln Glu Ala Gln Ser Phe Ala Asn Glu Leu Leu
        115                 120                 125
```

Ser Gln Gln Gln Ala Ala Leu Asn Gly Trp Lys Arg Ala Leu Ser Thr
    130                 135                 140

Leu Asp Ser Ile Pro Ala Arg Lys Asp Phe Pro Phe Leu Gly Arg Pro
145                 150                 155                 160

Ser Thr Pro Thr Lys Gly Arg Gly Gln Ala Met Gly Thr Leu Gln Asp
                165                 170                 175

Tyr Val Asp Thr Ala Glu Val Gln Arg Ala Gly Ser Glu Ala Pro Glu
            180                 185                 190

Ile Leu Ala Arg Phe Ala His Gln Val Ser Thr Ile Glu Lys Asn Val
        195                 200                 205

Ser Glu Ile Ala Thr Asp Thr Gln Arg Leu Val Asp Glu Ala Phe Pro
    210                 215                 220

Thr Ser Val Glu Pro Val Asp Gly Leu Leu Gln Glu Met Glu Thr Ile
225                 230                 235                 240

Ser Asn Lys Ile Gln Ser Asp Tyr Glu His Val Leu Ala Leu Pro Asn
                245                 250                 255

Asn Pro Lys Thr Leu Ala Asn Val Ser Arg Leu Ala Leu Ser His Thr
            260                 265                 270

Gln Asp Leu Leu Pro Ser Leu Leu Asp Val Ser Asn Glu Ile Gln Ala
        275                 280                 285

Gly Gln Glu Glu Ala Val Arg Arg Tyr Asn Ala Ala Val Lys Ala Ser
    290                 295                 300

Ser Ser His Met Arg Leu Ile Ser Ser Ile Glu Tyr Arg Leu Ala Glu
305                 310                 315                 320

Thr Leu Pro Gln Ile Asn Ser Leu Pro Leu Gln Ser Glu Ala Phe Asp
                325                 330                 335

Val Ile Tyr Thr Val Phe His Met Pro Ile Ile Tyr Gly Ser Val Leu
            340                 345                 350

Ile Glu Ser Val Arg Arg Arg Glu Phe Asn Asp Lys Met Lys Asn Asp
        355                 360                 365

Ser Leu Thr Leu Ala Glu Glu Met Ser Val Phe Arg Glu Glu Glu Gln

370 375 380

Arg Arg Arg Lys Lys Trp Leu Lys Asn Met Ala Asp Phe Ile Ser Ile
385 390 395 400

Ser Asp Ala Thr Thr Pro Gly Ile Glu Ile Asn Leu Arg Gly Gln Glu
405 410 415

Gln Glu Trp Pro Ala Val Ser Arg Lys Glu Val Glu Ser Tyr Ile Glu
420 425 430

Asp Leu Lys Asn Lys Pro Asp Met Glu Asn His Ala Gln Glu Leu Met
435 440 445

Gln Arg Phe Lys Glu Leu Asp Ala Pro Thr Arg Met Gln Arg Arg Arg
450 455 460

Ala Lys Ala Phe Lys Gln Gly Ser Leu Phe Asp Leu Ser Arg Ser Ser
465 470 475 480

Leu Leu Leu Arg Ser Asp Asp Met Val Arg Ser Leu Lys Asp Glu Lys
485 490 495

Val Lys Leu Glu Asp Arg Leu Lys Gly Ser Glu Ser Arg Ile Arg Lys
500 505 510

Leu Glu Asp Leu Leu His Arg Gln Ser His Met Ser Arg Pro Ser Ser
515 520 525

Gly Asn Phe Ser Leu Asp Phe Pro Ser Ser Pro Ala Ser Pro His Pro
530 535 540

Asp Ser Leu Ser Arg Arg Ser Ser Val Ser Ser Arg Arg Met Ser Ala
545 550 555 560

Asn Gln Ala Pro Glu Glu Lys Ala Leu Val Gln Arg Ile Thr Arg Leu
565 570 575

Glu Ala Glu Leu Ala Ala Glu Arg Asp Thr Val Gln Arg Leu Gln Lys
580 585 590

Glu Ala His Ala Glu Arg Gln Ser Asn Thr Asp Lys Ile Gln Glu Ala
595 600 605

Gln Ser Thr Lys Lys Asp Leu Ile Gly Asn Leu Glu Ala Arg Gln Arg
610 615 620

```
Glu Phe Asp Asp Glu Arg Arg Phe Leu Glu Gly Glu Leu Arg Arg Leu
625                 630                 635                 640

Arg Ile Arg Thr Glu Glu Leu Glu Glu Glu Val Asp Arg Asn Leu Asp
                645                 650                 655

Ser Arg Glu His Glu Lys Gln Asp Ala Asp Glu Arg Ile His His Leu
            660                 665                 670

Glu Met Glu Leu Gln Asp Val His Ala Arg Ala Glu Glu Glu Leu Arg
        675                 680                 685

Lys Ala Asn Glu Thr Ile Gly His Leu Gln Ser Glu Lys Gly Val Gly
    690                 695                 700

Glu Thr Leu Arg Ala Arg Ile Glu Glu Leu Gln Asn Gln Val Ala Glu
705                 710                 715                 720

Asn Glu Arg Lys Glu Asn Asp Asn Lys His Ala Leu Gln Val Ala Phe
                725                 730                 735

Leu Asn Leu Ser Pro Gly Gly Ser Val Pro Thr Glu Phe Pro Asn Ile
            740                 745                 750

Ile Lys Ala Ile Glu Val Leu Ser Glu Gly Leu Thr Ile His Ala Lys
        755                 760                 765

Asn Ala Glu Glu Ser Ala Ala Lys Ala Ser Ala Glu Ser Lys Val Leu
    770                 775                 780

Glu Glu Arg Leu Gly Leu Met Glu Ser Glu Leu Glu Asp Leu Arg Lys
785                 790                 795                 800

Thr Ala Thr Ser Arg Glu Thr Glu Leu Ser Gln Ala Arg Asn Glu Leu
                805                 810                 815

Ala Glu Gly Lys Ala Lys Leu Ser Ile Val Thr Ser Glu Leu Glu Asp
            820                 825                 830

Glu Arg Ser Lys Leu Ile Ala Leu Gln Ser Gln Phe Ala Ala Gly Glu
        835                 840                 845

Thr Gly Ser Asp Ala Leu Arg Glu Arg Val Thr Glu Glu Glu Arg Lys
    850                 855                 860

Leu Thr Asp Leu Ala Gln Arg Leu Ala Glu Val Glu Thr Leu Ala Arg
865                 870                 875                 880
```

Gln Ser Glu Glu Glu Val Thr Thr Trp Lys Lys Lys Val Glu Ala Met
885 890 895

Ser Glu Ala Glu Gln Gln Thr Thr Val Arg Phe Glu Thr Cys Gly Val
900 905 910

Arg Phe Gln Glu Leu Ser Lys Gln Leu Phe Ala Gln Val Glu Lys Leu
915 920 925

Glu Arg Met Leu Glu Gln Leu Gly Phe Thr Ile Val Arg Gln Asn Gly
930 935 940

Asp Ile Val Val Gln Arg Ser Ser Lys Val Asn Ala Leu Ser Ser Thr
945 950 955 960

Gly Asp Ser Leu Ser Gln Ser Gly Val Val Ser Val Arg Pro Asp Ala
965 970 975

Ser Leu Leu Asp Trp Met His Ala Asp Ser Lys Glu Glu Glu Thr Asp
980 985 990

Lys Phe Met Ala Phe Val Glu Ser Leu Tyr Gln Phe Asp Val Asp Ile
995 1000 1005

Phe Gly Asp Ala Val Val Lys Arg Val Lys Asp Ile Glu Val Leu
1010 1015 1020

Ala Arg Lys Trp Gln Lys Glu Ala Arg Gly Tyr Arg Asp Lys Tyr
1025 1030 1035

His Arg Val Gln Ser Glu Ala His Asp Lys Ile Ala Tyr Arg Ser
1040 1045 1050

Phe Lys Glu Gly Asp Leu Ala Leu Phe Leu Pro Thr Arg Asn Gln
1055 1060 1065

Ala Ile Arg Ser Trp Ala Ala Phe Asn Val Gly Ala Pro His Tyr
1070 1075 1080

Phe Leu Arg Glu Gln Asp Ala His Lys Leu Gln Thr Arg Asp Trp
1085 1090 1095

Leu Leu Ala Arg Ile Thr Lys Ile Glu Glu Arg Val Val Asp Leu
1100 1105 1110

Ser Lys Ser Met Asn Gly Ala Asn Pro Asp Arg Arg Ser Ile Gly
1115 1120 1125

Glu Val Ser Asp Gly Thr Ser Leu Asp Asp Glu Asn Pro Phe Glu
1130 1135 1140

Leu Ser Asp Gly Leu Arg Trp Tyr Leu Leu Asp Ala Thr Glu Glu
1145 1150 1155

Lys Pro Gly Ala Pro Ala Thr Pro Gly Leu Gly Lys Ser Thr Val
1160 1165 1170

Ala Pro Ala His Val Asp Ala Lys Gly Ser Ile Arg Met Lys Arg
1175 1180 1185

Thr Ser Ala Gly Gly Asn Ile Ala Lys Thr Leu Thr Lys Ser Leu
1190 1195 1200

Asp Ser Arg Arg Asn Ser Ser Asn Ser Lys Lys Gly His Ser Ser
1205 1210 1215

Thr Pro Ser Gln Arg Gly Asn Glu Ser Thr Thr Asp Leu Ala Arg
1220 1225 1230

Leu Ala Glu Gln Asp Thr Ala Ala Ser Leu His Ser Arg Glu Ala
1235 1240 1245

Ala Pro Lys Pro Asp Glu Ile Leu Glu Ile Phe Ala Val Gly Arg
1250 1255 1260

Thr Glu Tyr Lys Ala Ser Leu Gln Val Glu Pro Lys Leu Asp Ala
1265 1270 1275

Leu Trp Asn Glu Arg Ser Pro Ala Pro Glu Cys Arg Ala Asn Glu
1280 1285 1290

Ala Val Asn Tyr Ala Lys Lys Gly Glu
1295 1300

<210> 3
<211> 30
<212> DNA
<213> Artificial Sequence

<220>
<223> Primer 3atg11F

<400> 3
actagttcta gagttgagac attggctcgt

<210> 4
<211> 29
<212> DNA

<213> Artificial sequence

<220>
<223> Primer 3atg11R

<400> 4
gaattcgcta gccgtccaga gacctaggg 29

<210> 5
<211> 24
<212> DNA
<213> Artificial sequence

<220>
<223> Primer 5atg11F

<400> 5
gcggccgcgg tcggcgttcg aagg 24

<210> 6
<211> 30
<212> DNA
<213> Artificial sequence

<220>
<223> Primer 5atg11R

<400> 6
tactagtggg ggtatcgaag tcttcggggc 30

<210> 7
<211> 30
<212> DNA
<213> Artificial sequence

<220>
<223> Primer PE001-F

<400> 7
cggatccacc atgcgtctca ctctattatc 30

<210> 8
<211> 28
<212> DNA
<213> Artificial sequence

<220>
<223> Primer PE001-R

<400> 8
accacgtgtc aaaactgcca cacgtcgt 28

<210> 9
<211> 28
<212> DNA
<213> Artificial sequence

<220>

<223> Primer hacA1

<400> 9
tttgctagcc gacggggatg ctttttgc 28

<210> 10
<211> 33
<212> DNA
<213> Artificial sequence

<220>
<223> Primer hacA2

<400> 10
cttccatcat ggtggatcct tcaagcgtga cag 33

<210> 11
<211> 33
<212> DNA
<213> Artificial sequence

<220>
<223> Primer hacA3

<400> 11
ctgtcacgct tgaaggatcc accatgatgg aag 33

<210> 12
<211> 30
<212> DNA
<213> Artificial sequence

<220>
<223> Primer hacA4

<400> 12
ccagcgacgg acactgcagg atgttgtgtc 30

<210> 13
<211> 30
<212> DNA
<213> Artificial sequence

<220>
<223> Primer hacA5

<400> 13
gacacaacat cctgcagtgt ccgtcgctgg 30

<210> 14
<211> 25
<212> DNA
<213> Artificial sequence

<220>
<223> Primer hacA6

<400> 14

ttcacgtgat aaaattataa ggatt 25

<210> 15
<211> 20
<212> DNA
<213> Artificial sequence

<220>
<223> AMG forward primer

<400> 15
ttatacatgg actcgtgact 20

<210> 16
<211> 20
<212> DNA
<213> Artificial sequence

<220>
<223> AMG reverse primer

<400> 16
agaatcacag gcatcgcagg 20

<210> 17
<211> 20
<212> DNA
<213> Artificial sequence

<220>
<223> forward primer

<400> 17
agaagagaaa gtcatggggc 20

<210> 18
<211> 20
<212> DNA
<213> Artificial sequence

<220>
<223> reverse primer

<400> 18
agggcacttc cttctccttc 20